(19) Europäisches Patentamt
European Patent Office
Office européen des brevets

(11) **EP 4 606 372 A1**

(12) **EUROPEAN PATENT APPLICATION**
published in accordance with Art. 153(4) EPC

(43) Date of publication:
**27.08.2025 Bulletin 2025/35**

(21) Application number: **23879792.2**

(22) Date of filing: **17.10.2023**

(51) International Patent Classification (IPC):
*A61K 9/48* (2006.01)     *A61K 31/713* (2006.01)
*A61K 38/02* (2006.01)     *A61K 47/10* (2017.01)
*A61K 47/18* (2017.01)     *A61K 47/24* (2006.01)
*A61K 47/28* (2006.01)     *A61K 48/00* (2006.01)
*A61P 7/00* (2006.01)      *A61P 37/04* (2006.01)
*B01J 19/22* (2006.01)

(52) Cooperative Patent Classification (CPC):
**A61K 9/48; A61K 31/713; A61K 38/02;
A61K 47/10; A61K 47/18; A61K 47/24;
A61K 47/28; A61K 48/00; A61P 7/00; A61P 37/04;
B01J 19/22**

(86) International application number:
**PCT/JP2023/037566**

(87) International publication number:
**WO 2024/085149 (25.04.2024 Gazette 2024/17)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **18.10.2022 JP 2022167048**

(71) Applicant: **Tipton Corp.
Nagoya-shi, Aichi 457-8566 (JP)**

(72) Inventors:
• **NIWA, Takako**
  **Tokyo 103-8426 (JP)**
• **MORITA, Koji**
  **Tokyo 103-8426 (JP)**
• **KOBAYASHI, Tomoyuki**
  **Nagoya-shi Aichi 457-8566 (JP)**
• **KATO, Kairi**
  **Nagoya-shi Aichi 457-8566 (JP)**

(74) Representative: **Müller-Boré & Partner
Patentanwälte PartG mbB
Friedenheimer Brücke 21
80639 München (DE)**

(54) **TAYLOR REACTOR AND CAPSULE PARTICLE MANUFACTURING METHOD**

(57) An object of the present invention is to provide a capsule particle manufacturing method. A method of manufacturing a capsule particle by using a Taylor reactor is provided.

Fig. 1

EP 4 606 372 A1

## Description

TECHNICAL FIELD

The present disclosure relates to a Taylor reactor and a capsule particle manufacturing method.

BACKGROUND ART

A capsule particle can be indicated as a means for delivering an enclosure to a target. The capsule particle usually has an outer capsule portion enclosing contents inside. This can improve stability and deliver the contents to the target. Examples of utilizing a capsule particle include cosmetics, pharmaceuticals, functional foods, printing technologies, and industrial chemicals. As a method of manufacturing a capsule particle, various methods are known, such as a solvent injection method, a handshaking method, a reverse phase evaporation method, a membrane permeation pH gradient method, a bubble method, a microfluidization method, a thin film hydration method, a heating method, a freeze-thaw method, and a dehydration-rehydration method.

Pharmaceuticals using various nucleic acids such as plasmid DNA, antisense oligonucleic acid, siRNA, miRNA, and mRNA have been researched and developed. For example, an mRNA vaccine is a type of vaccine that expresses a protein of interest in a living body upon administration of mRNA into the living body, thereby inducing an immune reaction. Examples of antigenic proteins in mRNA vaccines include viral antigens and cancer antigens, and in recent years, there are examples of spike proteins of SARS-CoV-2. In addition, there is also an attempt to treat a genetic disease by administering mRNA encoding a normal protein.

Nucleic acids are macromolecules negatively charged when administered as they are, and thus cannot permeate the cell membrane. In particular, mRNA involves problems of being rapidly degraded in blood and inducing an inflammatory reaction in vivo. Therefore, various drug delivery systems are used for delivery of nucleic acids.

Drug delivery systems for delivering nucleic acids include, for example, a lipid nanoparticle. The lipid nanoparticle is predominantly spherical or sphere-like in shape. As the lipid, fatty acids, acylglycerols, waxes, and mixtures thereof with surfactants may be used. A stabilizer may be used, and as the stabilizer, phospholipids, biomembrane lipids such as sphingomyelin, bile salts, sterols (for example, cholesterol), and the like may be used. For the lipid nanoparticle, see, e.g., Shah et al., Lipid Nanoparticles: Production, Characterization and Stability, Springer, 2015.

Examples of a lipid component of the lipid nanoparticle include phospholipids, cholesterol, pH-responsive cationic lipids, and PEGylated lipids, in which nucleic acids are encapsulated.

A manufacturing flow for a lipid nanoparticle (LNP) in which a nucleic acid is encapsulated is generally as follows. First, a lipid ethanol solution and an aqueous nucleic acid solution (acidic) are brought into contact with each other, and the lipid solution and the nucleic acid solution are mixed (mixing step). Then, if necessary, the mixed liquid is diluted (dilution step). Next, ultrafiltration or diafiltration is performed, and concentration is performed (purification step). At this stage, the free nucleic acid is removed, the solvent (ethanol) is removed, substitution to a final medium occurs, and the lipid nanoparticle is concentrated to a target concentration. Next, filter filtration is performed.

As a conventional method of mixing a lipid component and a nucleic acid component, a dropping mixing method, an in-line mixing method, and a microchannel mixing method are known. The dropping mixing method involves problems of unsuitability for continuous production because it is a batch method, and of non-uniformity of quality of the obtained particle. The in-line mixing enables continuous production, but involves use of a flow path on the order of millimeters, and therefore, the obtained particle has a large size, non-uniform quality, and poor reproducibility. The microchannel mixing method can control the particle size to be small and provides high reproducibility, but, on the other hand, a low production amount because a flow path is as small as about 0.1 mm.

There is a need for a capsule particle manufacturing method suitable for large-scale production.

CITATIONS LIST

NON-PATENT LITERATURE

Non Patent Literature 1: Shah et al., Lipid Nanoparticles: Production, Characterization and Stability, Springer, 2015

SUMMARY OF INVENTION

TECHNICAL PROBLEMS

An object of the present inventors is to provide a capsule particle manufacturing method that at least partially solves the problems of conventional particle manufacture.

SOLUTIONS TO PROBLEMS

As a result of intensive studies to solve the above problems, the present inventors have found that a capsule particle can be manufactured by using a Taylor reactor, as an example, and have completed the present invention including this as an embodiment.

The present disclosure includes the following embodiments.

[1] A capsule particle manufacturing method using a Taylor reactor.
[2] The manufacturing method according to Embodiment 1, wherein

the Taylor reactor includes an outer cylinder, an inner cylinder, and one or more inlets,
the inner cylinder is rotatably disposed in the outer cylinder and forms a reaction chamber having an annular shape between the inner cylinder and the outer cylinder, and
at least one of the inlets is disposed so as to allow a fluid to flow into the reaction chamber.

[3] The manufacturing method according to Embodiment 2, wherein

the one or more inlets include a first inlet and a second inlet, and the first inlet and the second inlet are independent of each other and are each connected to the reaction chamber,
a particle-forming component is injected from the first inlet into the reaction chamber,
an enclosed component is injected from the second inlet into the reaction chamber, and
the inner cylinder is rotated to mix the particle-forming component and the enclosed component in the reaction chamber.

[4] The manufacturing method according to Embodiment 2, wherein

the one or more inlets include a first line and a second line, the first line and the second line are disposed so as to merge, and a merged line is connected to the reaction chamber via a common inlet, and
a particle-forming component is injected from the first line, an enclosed component is injected from the second line, the particle-forming component and the enclosed component are mixed in front of the reaction chamber to generate a particle-forming component-enclosed component mixed liquid, then the particle-forming component-enclosed component mixed liquid is injected into the reaction chamber via the common inlet, the inner cylinder is rotated, and the particle-forming component-enclosed component mixed liquid is further mixed in the reaction chamber.

[5] The manufacturing method according to Embodiment 3 or 4, wherein

the particle-forming component contains a polymer component or a lipid component, and
the enclosed component contains a nucleic acid, a protein, a polypeptide, a peptide, or a low molecular weight compound.

[6] The manufacturing method according to Embodiment 5, wherein

the particle-forming component contains a lipid component, and
the enclosed component contains a nucleic acid.

[7] The manufacturing method according to Embodiment 3, wherein the particle-forming component is injected first, and then the enclosed component is injected.
[8] The manufacturing method according to Embodiment 3, wherein the enclosed component is injected first, and then the particle-forming component is injected.
[9] The manufacturing method according to Embodiment 3, wherein the particle-forming component and the enclosed component are injected simultaneously.
[10] The manufacturing method according to any one of Embodiments 2 to 9, wherein a peripheral speed of the inner cylinder is 0.5 to 47 m/s.
[11] The manufacturing method according to any one of Embodiments 2 to 10, wherein a gap width between the inner cylinder and the outer cylinder is 0.01 mm to 5 mm.

[12] The manufacturing method according to any one of Embodiments 1 to 11, wherein a stirring time for performing a Taylor reaction is 1 minute or shorter.

[13] The manufacturing method according to any one of Embodiments 3 and 5 to 12, wherein a ratio of an injection rate of the particle-forming component to an injection rate of the enclosed component is a ratio selected from a range of 1:1 to 1:9.

[14] The manufacturing method according to any one of Embodiments 3 to 13, wherein inner diameters of the first inlet and the second inlet are the same or substantially the same.

[15] The manufacturing method according to any one of Embodiments 3 to 13, wherein inner diameters of the first inlet and the second inlet are different from each other.

[16] A Taylor reactor for use in a capsule particle manufacturing method, the Taylor reactor including an outer cylinder, an inner cylinder, and one or more inlets, wherein

the inner cylinder is rotatably disposed in the outer cylinder and forms a reaction chamber having an annular shape between the inner cylinder and the outer cylinder, and
at least one of the inlets is disposed so as to allow a fluid to flow into the reaction chamber.

[17] The reactor according to Embodiment 16, wherein

the one or more inlets include a first inlet and a second inlet, and the first inlet and the second inlet are independent of each other and are each connected to the reaction chamber,
the first inlet is for injecting a particle-forming component,
the second inlet is for injecting an enclosed component, and
the inner cylinder is rotated for mixing the particle-forming component and the enclosed component in the reaction chamber.

[18] The reactor according to Embodiment 16, wherein

the one or more inlets have a first line and a second line,
the first line is for injecting a particle-forming component,
the second line is for injecting an enclosed component,
the first line and the second line merge in front of the reaction chamber,
a downstream side of a merging portion forms a particle-forming component-enclosed component mixed liquid line, which is then connected to the reaction chamber via a common inlet, and
the inner cylinder is rotated for further mixing a particle-forming component-enclosed component mixed liquid in the reaction chamber.

[19] The reactor according to Embodiment 17 or 18, wherein

the particle-forming component contains a polymer component or a lipid component, and
the enclosed component contains a nucleic acid, a protein, a polypeptide, a peptide, or a low molecular weight compound.

[20] The reactor according to Embodiment 19, wherein

the particle-forming component contains a lipid component, and
the enclosed component contains a nucleic acid.

[21] The reactor according to Embodiment 17 or Embodiment 19 dependent on Embodiment 17, wherein, when a side on which a liquid is supplied to the reaction chamber is defined as an upstream side and a side on which the liquid is discharged from the reaction chamber is defined as a downstream side, the first inlet is disposed on the upstream side and the second inlet is disposed on the downstream side.

[22] The reactor according to Embodiment 17 or Embodiment 19 dependent on Embodiment 17, wherein, when a side on which a liquid is supplied to the reaction chamber is defined as an upstream side and a side on which the liquid is discharged from the reaction chamber is defined as a downstream side, the second inlet is disposed on the upstream side and the first inlet is disposed on the downstream side.

[23] The reactor according to Embodiment 17 or Embodiment 19 dependent on Embodiment 17, wherein the first inlet and the second inlet are disposed on the same cross section perpendicular to an axial direction of the inner cylinder.

[24] The reactor according to any one of Embodiments 16 to 23, wherein a gap width between the inner cylinder and the outer cylinder is 0.01 mm to 5 mm.

[25] The reactor according to any one of Embodiments 17 and 19 to 24, wherein a ratio of an injection rate of the particle-forming component to an injection rate of the enclosed component is a ratio selected from a range of 1:1 to 1:9.

[26] The reactor according to any one of Embodiments 17 to 25, wherein inner diameters of the first inlet and the second inlet are the same or substantially the same.

[27] The reactor according to any one of Embodiments 17 to 25, wherein inner diameters of the first inlet and the second inlet are different from each other.

The present specification includes the disclosure of Japanese Patent Application No. 2022-167048 on which priority of the present application is based.

ADVANTAGEOUS EFFECTS OF INVENTION

As an effect of the present disclosure, a capsule particle can be manufactured.

BRIEF DESCRIPTION OF DRAWINGS

Fig. 1 is a schematic diagram of a Taylor reactor.

Fig. 2 shows an arrangement of an inlet including a first line and a second line.

Fig. 3 shows an arrangement of the first inlet and the second inlet.

Fig. 4 shows an arrangement of an inlet including a first line, a second line, and a third line.

Fig. 5 shows an arrangement of a first inlet including a first line and a second line, and a second inlet. Fig. 5 corresponds to an A-A' cross section in Fig. 1.

Fig. 6 shows an arrangement of a first inlet, a second inlet, and a third inlet.

Fig. 7 is a diagram showing plasma iron concentrations in mice before administration of TfR2 siRNA-enclosed nucleic acid lipid particles.

Fig. 8 is a diagram showing the plasma iron concentrations in the mice 1 day after administration of the TfR2 siRNA-enclosed nucleic acid lipid particles.

Fig. 9 is a diagram showing the plasma iron concentrations in the mice 7 days after administration of the TfR2 siRNA-enclosed nucleic acid lipid particles.

Fig. 10 is a diagram showing HA-specific IgG concentrations in serum in mice after administration of A_Sin_GP1908_2015_H1 mRNA-enclosed nucleic acid lipid particles (Reference Example 2, Specimen 9, Specimen 10, and Specimen 11).

Fig. 11 is a diagram showing the HA-specific IgG concentrations in serum in mice after administration of A Sin_GP1908_2015_H1 mRNA-enclosed nucleic acid lipid particles (Reference Example 3, Reference Example 4, Specimen 12, Specimen 13, and Specimen 14).

DESCRIPTION OF EMBODIMENTS

In a certain embodiment, the present disclosure provides a capsule particle manufacturing method using a Taylor reactor.

The capsule particle refers to a particle in which contents are encapsulated. In the present specification, the contents to be enclosed in the capsule particle are sometimes referred to as enclosed component (component to be enclosed). In addition, in the present specification, a component surrounding the contents to be enclosed is sometimes referred to as component forming a particle (particle-forming component) or capsule-forming component. In the capsule particle, the enclosed component and the particle-forming component may form a complex. The capsule particle includes lipid particles and polymer particles, but is not limited thereto. In a certain embodiment, the capsule particle may be a particle having a mean particle diameter of 10 nm or more, 20 nm or more, 30 nm or more, 40 nm or more, 50 nm or more, 60 nm or more, 70 nm or more, 80 nm or more, 90 nm or more, 100 nm or more, 1 $\mu$m or more, 10 $\mu$m or more, 100 $\mu$m or more, for example, 1 mm or more, and 2 mm or less, 1 mm or less, 100 $\mu$m or less, 10 $\mu$m or less, 1 $\mu$m or less, for example, 100 nm or less, 90 nm or less, 80 nm or less, 70 nm or less, 60 nm or less, 50 nm or less, 40 nm or less, 30 nm or less, 20 nm or less, for example, 10 nm or less, but is not limited thereto. The capsule particle may contain any contents (enclosed component). In addition, the size and mean particle diameter of the capsule particles can be appropriately adjusted depending on the particle-forming component and the enclosed component. For example, the particle diameter may vary depending on the particle-forming component, the enclosed component, and a combination thereof.

In a certain embodiment, the particle-forming component may include a polymer component, a lipid component, an auxiliary component, and the like. Examples of the polymer component include biodegradable polymers. Such biodegradable polymers include polylactic acid, polyglycolic acid, a polylactic acid-glycolic acid copolymer (PLGA), and a polydepsipeptide-lactic acid copolymer, but are not limited thereto. Examples of the lipid component include amphoteric lipids, neutral lipids, and cationic lipids. The lipid component specifically includes phospholipids, sphingolipids, glycolipids, glycerolipids, sterol lipids, and polymer complex lipids, but is not limited thereto. In a certain embodiment, the particle-forming component can be used by being dissolved or the like. Examples of the enclosed component include nucleic acids, proteins, polypeptides, peptides, low molecular weight compounds such as drug compounds, cosmetics or cosmetic components, pigments, skin care components, and supplement components, but are not limited thereto. In a certain embodiment, the enclosed component can be used by being dissolved or the like.

In the present specification, unless otherwise specified, the mean particle diameter refers to mean volume diameter as determined by measurement and calculation based on the principle of dynamic light scattering. Here, it is assumed that there are n1, n2, ... ni, ... nk particles having a particle diameter of d1, d2, ... di, ... dk in ascending order of particle diameters. A volume per particle is defined as vi. At this time, the mean volume diameter is determined by:

[Mathematical formula 1]

$$MV = \Sigma(Vi \cdot di)/\Sigma(di).$$

The polymer particle refers to a particle containing a polymer material. In a certain embodiment, the polymer particle may be a particle having a mean particle diameter of 10 nm or more, 20 nm or more, 30 nm or more, 40 nm or more, 50 nm or more, 60 nm or more, 70 nm or more, 80 nm or more, 90 nm or more, 100 nm or more, 1 $\mu$m or more, 10 $\mu$m or more, 100 $\mu$m or more, for example, 1 mm or more, and 2 mm or less, 1 mm or less, 100 $\mu$m or less, 10 $\mu$m or less, 1 $\mu$m or less, 500 $\mu$m or less, 400 $\mu$m or less, 300 $\mu$m or less, 200 $\mu$m or less, for example, 100 nm or less, 90 nm or less, 80 nm or less, 70 nm or less, 60 nm or less, 50 nm or less, 40 nm or less, 30 nm or less, 20 nm or less, for example, 10 nm or less, but is not limited thereto. In a certain embodiment, the polymer particle may be a particle having a mean particle diameter of 10 nm to 500 $\mu$m, such as 20 nm to 400 $\mu$m, 30 nm to 300 $\mu$m, 20 nm to 200 $\mu$m, such as 10 nm to 100 $\mu$m, but is not limited thereto. Examples of the polymer particle include particles containing a biodegradable polymer as the particle-forming component. Examples of the biodegradable polymer include polymers containing polylactic acid. Examples of the polymer containing polylactic acid include a polylactic acid-glycolic acid copolymer (PLGA) and a polydepsipeptide-lactic acid copolymer, but are not limited thereto. The polymer particle may contain any contents (enclosed component).

The lipid particle refers to a particle containing a lipid component. The structure of the lipid particle is not particularly limited, and may be a lipid multilayer, a lipid bilayer, or a lipid monolayer, and may partially have a lipid multilayer, a lipid bilayer, or a lipid monolayer. Examples of the lipid particle include particles having a lipid bilayer, for example, liposomes or niosomes having a lipid bilayer, particles having a lipid monolayer, for example, lipid monolayer particles containing a tetraether lipid, particles partially having a lipid monolayer and/or a lipid bilayer, particles having a lipid multiayer, and lipid nanoparticles (LNPs), but are not limited thereto. The lipid particle may contain any contents (enclosed component), and examples of the enclosed component include a nucleic acid, a protein, a peptide, a low molecular weight compound, and a drug compound, and preferably include a nucleic acid, and more preferably include messenger RNA (mRNA), double-stranded RNA (siRNA), double-stranded RNA (dsRNA), double-stranded DNA (dsDNA), and single-stranded DNA (ssDNA), and further preferably include mRNA (a lipid particle containing a nucleic acid as the enclosed component is sometimes particularly referred to as nucleic acid lipid particle). The liposome refers to a lipid particle having at least one lipid bilayer. The liposome includes small unilamellar vesicles (SUVs), large unilamellar vesicles (LUVs), multilamellar vesicles (MLVs), oligolamellar vesicles (OLVs), medium unilamellar vesicles (MUVs), giant unilamellar vesicles (GUVs), and giant multilamellar vesicles (GMVs). The LUV can generally have a particle diameter of 100 nm or more, but may be classified as a particle having a particle diameter of 50 nm or more. The SUV can generally have a particle diameter of 20 to 100 nm, but may be classified as a particle having a particle diameter of less than 50 nm. Any classification may be adopted, but the same classification shall be used for both the LUV and the SUV. In the MLV, each vesicle has two or more bilayers. A separate aqueous compartment may be formed within the MLV. The liposome can have a double membrane composed of a phospholipid. The niosome can have a double membrane composed of a nonionic surfactant. See, e.g., JP 2013-536803 A. The description of liposomes is incorporated herein by reference.

The lipid component is roughly classified into an amphoteric lipid, a neutral lipid, an anionic lipid, and a cationic lipid. The lipid component is not particularly limited, and includes, but is not limited to, phospholipids, sphingolipids, glycolipids, glycerolipids, sterol lipids, and polymer complex lipids.

The cationic lipid includes lipids in which lipid molecules have a net positive charge regardless of pH, and lipids in which some lipid molecules have a net positive charge depending on pKa of the lipid at a selected pH, such as a physiological pH (sometimes referred to as pH-responsive cationic lipids). Examples of the lipid in which lipid molecules have a net positive charge regardless of pH include, but are not limited to, N,N-dioleyl-N,N-dimethylammonium chloride (DODAC), N,N-distearyl-N,N-dimethylammonium bromide (DDAB), N-[1-(2,3-dioleoyloxy)propyl]-N,N,N-trimethylammonium chloride (DOTAP), N,N-dioctadecylamidoglyceryl spermine (DOGS), N-[1-(2,3-dioleyloxy)propyl]-N,N,N-trimethylammonium chloride (DOTMA), 2,3-dioleyloxy-N-[2(spermine carboxamide)ethyl]-N,N-dimethyl-1-propanaminam trifluoroacetate (DOSPA), N-[1-(2,3-dimyristyloxy)propyl]-N,N-dimethyl-N-(2-hydroxyethyl)ammonium bromide (DMRIE), and 3β-N-(N',N',-dimethylaminoethane)-carbamoylcholesterol (DC-Chol). Examples of the pH-responsive cationic lipid include, but are not limited to, 1,2-dioleoyloxy-3-dimethylaminopropane (DODAP), N,N-dimethyl-(2,3-dioleyloxy)propylamine (DODMA), 1,2-dilinoleyloxy-3-dimethylaminopropane (DLinDMA), 2,2-dilinoleyl-4-(2-dimethylaminoethyl)-[1,3]-dioxolane (DLin-KC2-DMA), and heptatriaconta-6,9,28,31-tetraene-19-yl-4-(dimethylamino)butanoate (DLin-MC3-DMA). See, e.g., WO 2015/005253 and WO 2021/060440. The description of cationic lipids is incorporated herein by reference.

The amphiphilic lipid refers to a lipid having both a hydrophilic group and a hydrophobic group. Examples of the amphiphilic lipid include, but are not limited to, ionic lipids, phospholipids, sphingolipids, glycolipids, and glycerolipids. Examples of the phospholipid include glycerophospholipids and sphingophospholipids. Examples of the glycerophospholipid include phosphatidylcholine, phosphatidylethanolamine, phosphatidylserine, phosphatidylinositol, and phosphatidylcholine. Examples of the sphingophospholipid include sphingomyelin and sphingoethanolamine. Examples of the glycolipid include glyceroglycolipids and sphingoglycolipids. Examples of the glyceroglycolipid include monogalactosyldiacylglycerol, digalactosyldiacylglycerol, and sulfoquinobosyldiacylglycerol. Examples of the sphingoglycolipid include ceramide and ganglioside.

Specific examples of the phospholipid include, but are not limited to, 1,2-dioleoyl-sn-glycero-3-phosphocholine (DOPC), 1,2-dimyristoyl-sn-glycero-3-phosphocholine (DMPC), 1,2-distearoyl-sn-glycero-3-phosphocholine (DSPC), 1,2-dioleoyl-sn-glycero-3-phosphoethanolamine (DOPE), 1,2-diacyl-sn-glycero-3-phosphocholine (PC), 1, 2-diacyl-sn-glycero-3-phosphatidylethanolamine (PE), 1,2-diacyl-sn-glycero-3-phosphatidylserine (PS), 1,2-diacyl-sn-glycero-3-phosphatidylglycerol (PG), 1,2-diacyl-sn-glycero-3-phosphatidic acid (PA), 1,2-didecanoyl-sn-glycero-3-phosphocholine (DDPC), 1,2-diurauroyl-sn-glycero-3-phosphocholine (DLPC), 1,2-dilinoleoyl-sn-glycero-3-phosphocholine (DPPC), 1,2-dierythroyl-sn-glycero-3-phosphocholine (DLoPC), 1,2-dierucoyl-sn-glycero-3-phosphocholine (DEPC), 1-myristoyl-2-palmitoyl-sn-glycero-3-phosphocholine (MPPC), 1-myristoyl-2-stearoyl-sn-glycero-3-phosphocholine (MSPC), 1-palmitoyl-2-myristoyl-sn-glycero-3-phosphocholine (PMPC), 1-palmitoyl-2-stearoyl-sn-glycero-3-phosphocholine (PSPC), 1-palmitoyl-2-oleoyl-sn-glycero-3-phosphocholine (POPC), and 1-stearoyl-2-oleoyl-sn-glycero-3-phosphocholine (SOPC). See, e.g., WO 2021/060440. The description of phospholipids is incorporated herein by reference.

Examples of the sterol lipid include animal-derived sterol, plant-derived sterol, and microorganism-derived sterol. Examples of the animal-derived sterol include cholesterol, cholesterol succinic acid, dihydrocholesterol, 7-dehydrocholesterol, lanosterol, dihydrolanosterol, and desmosterol. Examples of the plant-derived sterol include brassicasterol, campesterol, stigmasterol, and sitosterol. Examples of the microorganism-derived sterol include timosterol and ergosterol.

The polymer composite lipid refers to a lipid to which a nonionic polyether, a nonionic polyester, a nonionic polyamino acid, or a nonionic polypeptide, or a polymer in which a terminal thereof is alkoxylated, or the like is added. The polymer added to the polymer composite lipid is not particularly limited, but is preferably a nonionic polyether or a nonionic polyester, or a polymer in which a terminal thereof is alkoxylated, more preferably a nonionic polyether or a nonionic monoalkoxy polyether, even more preferably a polyalkylene glycol or a monomethoxy polyalkylene glycol, and still more preferably polyethylene glycol or monomethoxy polyethylene glycol. The polyoxyalkylenated lipid is a lipid modified with a polyalkylene glycol or a derivative thereof, and examples thereof include, but are not limited to, a polymethylene glycolated lipid, a polyethylene glycolated lipid (PEG lipid), a polypropylene glycolated lipid, a polytetramethylene glycolated lipid, and a polyhexamethylene glycolated lipid. A weight average molecular weight of the polyalkylene glycol may be, for example, 200 to 10,000 Da. The PEG lipid includes, but is not limited to, PEG-cholesterol, PEG-phospholipid, PEG-ceramide, and PEG-diacylglycerol. A PEG moiety of the PEG lipid may, for example, have a molecular weight of 200 to 10,000 Da. The PEG moiety may be linear or branched. The modification with PEG can be performed by using a polyethylene glycol derivative such as stearylated polyethylene glycol, N-[carbonyl-methoxypolyethyleneglycol-1000]-1,2-dipalmitoyl-sn-glycero-3-phosphoethanolamine, N-[carbonyl-methoxypolyethyleneglycol-2000]-1,2-dipalmitoyl-sn-glycero-3-phosphoethanolamine, n-[carbonyl-methoxypolyethyleneglycol-5000]-1,2-dipalmitoyl-sn-glycero-3-phosphoethanolamine, N-[carbonyl-methoxypolyethyleneglycol-750]-1,2-distearoyl-sn-glycero-3-phosphoethanolamine, N-[carbonyl-methoxypolyethyleneglycol-1000]-1,2-distearoyl-sn-glycero-3-phosphoethanolamine, N-[carbonyl-methoxypolyethyleneglycol-2000]-1,2-distearoyl-sn-glycero-3-phosphoethanolamine, N-[carbonyl-methoxypolyethyleneglycol-5000]-1,2-distearoyl-sn-glycero-3-phosphoethanolamine, or 1,2-dimyristoyl-rac-glycero-3-methoxypolyethyleneglycol-2000 (PEG-DMG), but the present invention is not limited thereto. See, e.g., WO 2020/262150. The description of polyoxyalkylenated lipids is incorporated herein by reference.

The lipid component may be only one or more. In a certain embodiment, the lipid component can be a lipid containing a cationic lipid. In a further embodiment, the lipid component may include an amphiphilic lipid, a cationic lipid, a sterol lipid, and a polymeric complex lipid. In a further particular embodiment, contents of the respective lipids in the entire lipid component to be used can be a lipid containing 5 to 30 mol%, such as 12.5 mol%, such as 17.5 mol%, of a phospholipid as the amphiphilic lipid, 10 to 75 mol%, such as 30 to 70 mol%, such as 40 to 65 mol%, such as 45 mol%, such as 60 mol%, of a cationic lipid, 15 to 50 mol%, such as 21 mol%, such as 41 mol%, of cholesterol, and 1 to 10 mol%, such as 1 to 5 mol%, such as 1.5 mol%, of a polyoxyalkylenated lipid. See, e.g., WO 2009/127060 and WO 2015/005253. The description of a composition ratio of lipid components is incorporated herein.

Examples of the enclosed component (contents) contained in the capsule particle include, but are not limited to, a nucleic acid, a protein, a polypeptide, a peptide, a low molecular weight compound, and a drug compound. Examples of the nucleic acid include DNA and RNA. Examples of the RNA include, but are not limited to, messenger RNA (mRNA), double-stranded RNA (siRNA), single-stranded RNA (antisense oligo), small nuclear RNA (snRNA), non-coding RNA (ncRNA), long non-coding RNA (lncRNA), microRNA (miRNA), double-stranded RNA (dsRNA), and circular RNA. Examples of the DNA include, but are not limited to, a plasmid, single-stranded DNA, and double-stranded DNA. Examples of the low molecular weight compound include, but are not limited to, a drug compound, an anticancer agent, a compound acting on a living body, a substance having physiological activity, and a metabolite. Examples of the protein, polypeptide, or peptide include, but are not limited to, an antigenic protein, a therapeutic protein, a protein having physiological activity, and an antibody.

A combination of the particle-forming component and the enclosed component is not particularly limited. In a particular embodiment, the combination of the particle-forming component and the enclosed component may be selected in consideration of charge, polarity, and the like. In a certain embodiment, where the enclosed component is negatively charged, a positively charged component, for example, can be selected as the particle-forming component (e.g., lipid component). In a certain embodiment, where the enclosed component is positively charged, a negatively charged component, for example, can be selected as the particle-forming component (e.g., lipid component). In a certain embodiment, where the enclosed component is uncharged or little charged, an uncharged or little charged component, for example, can be selected as the particle-forming component (e.g., lipid component). In a certain embodiment, where the enclosed component is a component having high hydrophobicity, a medium for dissolving the enclosed component and a medium for dissolving the particle-forming component may be the same medium. In another embodiment, where the enclosed component is a component having high hydrophobicity, a medium for dissolving the enclosed component and a medium for dissolving the particle-forming component may be different media.

In a certain embodiment of the present invention, the capsule particle is a lipid particle (sometimes referred to as nucleic acid lipid particle) using a nucleic acid as the enclosed component. Examples of the nucleic acid lipid particle include a lipid nanoparticle (LNP) enclosing a nucleic acid and a liposome enclosing a nucleic acid. Examples of the lipid component used for the particle-forming component include an amphoteric lipid, a neutral lipid, a cationic lipid, an anionic lipid, an amphiphilic lipid, a sterol lipid, and a polymer complex lipid. These can be used alone or two or more thereof can be used in combination. When multiple lipids are used as the lipid component in the nucleic acid lipid particle, from the viewpoint of drug delivery in vivo, it is preferable to use a cationic lipid, an amphiphilic lipid, a sterol lipid, and a polymer composite lipid, and it is more preferable to use a pH-responsive cationic lipid, a phospholipid, cholesterol, and a PEG lipid. The contents of the respective lipids in the entire lipid component to be used can be appropriately adjusted depending on the type of lipid to be used, the nucleic acid to be enclosed, and the like, and can be, for example, 5 to 30 mol%, for example, 12.5 mol%, for example, 17.5 mol% for a phospholipid, 10 to 75 mol%, for example, 30 to 70 mol%, for example, 40 to 65 mol%, for example, 45 mol%, for example, 60 mol% for a cationic lipid, 15 to 50 mol%, for example, 21 mol%, for example, 41 mol% for cholesterol, and 1 to 10 mol%, for example, 1 to 5 mol%, for example, 1.5 mol% for a PEGylated lipid. The nucleic acid used as the enclosed component is not particularly limited, but is preferably messenger RNA (mRNA), double-stranded RNA (siRNA), double-stranded RNA (dsRNA), double-stranded DNA (dsDNA) and single-stranded DNA (ssDNA), and more preferably mRNA. When a cationic lipid is used as the lipid component, a ratio (N/P ratio) of a number of molecules (N) of the cationic lipid in the nucleic acid lipid particle to a number of phosphorus atoms (P) derived from the nucleic acid can be, for example, about 2.0 to 15.0, preferably about 2.0 to 12.0, more preferably about 2.0 to 9.0, and even more preferably about 3.0 to 9.0. A lower limit of the N/P ratio can be, for example, 2.0, preferably 2.5, and more preferably 3.0, and an upper limit thereof can be, for example, 15.0, preferably 12.0, and more preferably 9.0. A mean particle diameter of the nucleic acid lipid particles is not particularly limited, can be, for example, 1 nm to 1 μm, and is preferably 10 nm to 500 nm, more preferably 30 nm to 300 nm, even more preferably 50 nm to 200 nm, and most preferably 75 nm to 150 nm.

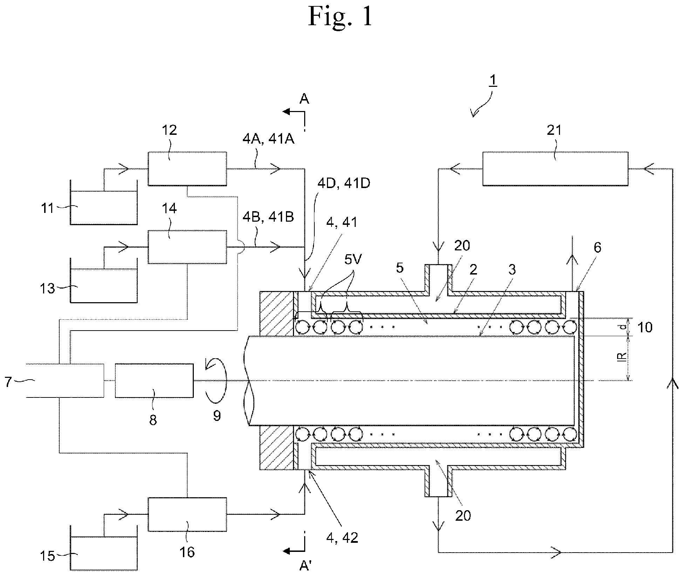

As shown in Fig. 1, a Taylor reactor 1 refers to a reactor that performs a Taylor reaction (also referred to as Taylor vortex flow reaction). In a certain embodiment, the Taylor reactor 1 has an inner cylinder 3 and an outer cylinder 2. A gap portion (d) is formed between an outer diameter (inner radius, IR) of the inner cylinder 3 and an inner diameter (outer radius, OR) of the outer cylinder 2, and is used as a reaction chamber 5 (also referred to as reaction tank) (OR = IR + d). Therefore, the Taylor reactor 1 includes the outer cylinder 2 and the inner cylinder 3, the inner cylinder 3 is rotatably disposed in the outer cylinder 2, and an annular reaction chamber 5 is formed between the inner cylinder 3 and the outer cylinder 2. The Taylor reactor 1 may have one or more inlets 4 (including a first inlet 41 and a second inlet 42) for allowing a liquid to flow into the reaction chamber 5. The inlet 4, the first inlet 41, and the second inlet 42 are sometimes referred to as injection openings or injection ports in the present specification. The Taylor reactor 1 may have at least one outlet 6 for allowing the liquid in the reaction chamber 5 to flow out to the outside. The outlet 6 is sometimes referred to as discharge opening or outflow port in the present specification. Reference numeral 7 denotes a controller that controls a rotation speed of a motor 8 that rotates the inner cylinder 3 and controls supply amounts of a first pump 12, a second pump 14, and a third pump 16. Reference numeral 20 denotes a jacket for cooling the reaction chamber 5, and reference numeral 21 denotes a chiller for controlling a jacket temperature by circulating a cooling medium in the jacket 20.

The enclosed component and the particle-forming component may react in solution to form a particle. Therefore, solvents for the enclosed component and the particle-forming component can be appropriately selected depending on properties of the respective components. In the manufacture of the nucleic acid lipid particle, for example, the nucleic acid and the lipid can be supplied to the reactor in a state in which the nucleic acid is dissolved in a citrate buffer and the lipid is dissolved in ethanol, respectively. Thus, the enclosed component and the particle-forming component can each be prepared in solution with the solvent and can each be subjected to the reactor.

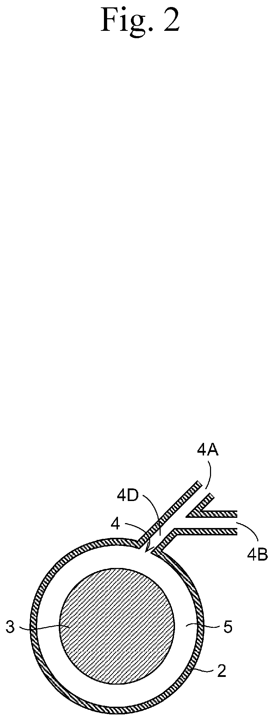

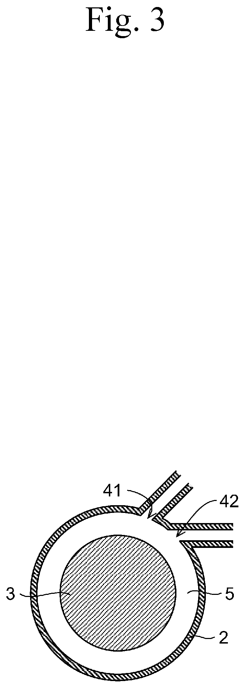

In a certain embodiment, the particle manufacturing method of the present disclosure may include a step of mixing a particle-forming component and an enclosed component. In the reaction chamber 5, in a cross-sectional view in a direction perpendicular to a rotation axis of the inner cylinder 3, a large number of sets of vortex rows 5V are formed in mutually opposite directions along the axial direction, and various solutions are mixed (Taylor vortex flow reaction) in a process in which the large number of vortex rows 5V move in the axial direction. In a particular embodiment, a solution of the particle-forming component is first supplied from a first line 4A via the first pump 12 from a first tank 11 containing the solution; a solution of the enclosed component is supplied from a second line 4B via the second pump 14 from a second tank 13 containing the solution; the solutions are mixed in a mixed liquid line 4D; and then the mixed liquid is supplied to the reaction chamber 5 of the Taylor reactor 1 via the inlet 4 (which is also the common inlet as claimed) (see Figs. 1 and 2). The mixing of the two liquids is performed before injection into the reaction chamber 5, and thus this is sometimes referred to as mixing in front of the reaction chamber (in front of the reaction tank), for convenience, in the present specification. In another embodiment, the particle-forming component and the enclosed component are supplied to the reaction chamber 5 of the Taylor reactor 1 via the respective independent injection ports (first inlet 41 and second inlet 42) without being brought into contact with each other, and then the two liquids are mixed for the first time in the reaction chamber 5 (see Fig. 3). The mixing of the two liquids is performed in the reaction chamber 5, and thus this is sometimes referred to as mixing "in the reaction chamber (in the reaction tank)", for convenience, in the present specification. The order of injecting the solution is not particularly limited, and the particle-forming component and the enclosed component may be injected simultaneously, the particle-forming component may be injected first, or the enclosed component may be injected first.

In another embodiment, the particle manufacturing method of the present disclosure can supply a mixing liquid containing the particle-forming component and the enclosed component mixed in advance to the reaction chamber 5 from the first inlet 41. In this case, the mixing of the particle-forming component and the enclosed component can take place outside the Taylor reactor 1. In addition, a diluting solution can be injected from the second inlet 42 different from the first inlet 41 through which the mixing liquid is injected (see Fig. 3). For example, when both the particle-forming component and the enclosed component are fat-soluble or hydrophobic, the particle-forming component and the enclosed component may be dissolved in the same solvent or different solvents and mixed. Such a pre-mixed liquid may then be used in the particle manufacturing method of the present disclosure.

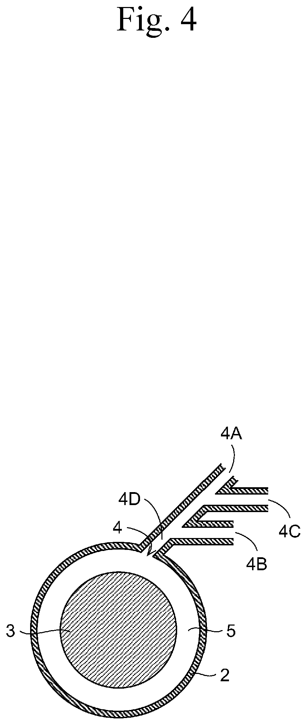

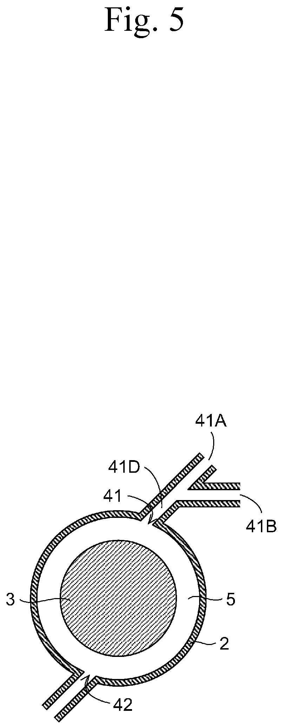

In a certain embodiment, the particle manufacturing method of the present disclosure may include a step of further injecting a diluting solution. The diluting solution may be injected at any stage. In a certain embodiment, the particle-forming component may first be supplied from the first line 4A, then the diluting solution may be supplied from a third line 4C, and they are mixed to generate a particle-forming component-diluting solution mixed liquid. Then, the enclosed component may be supplied from the second line 4B to the particle-forming component-diluting solution mixed liquid to generate a particle-forming component-enclosed component-diluting solution mixed liquid in the mixed liquid line 4D, which may be supplied to the reaction chamber 5 via the inlet 4 (which is also the common inlet as claimed) (see Fig. 4). In another embodiment, the enclosed component and the diluting solution may first be mixed to generate an enclosed component-diluting solution mixed liquid, and then the enclosed component-diluting solution mixed liquid may be mixed with the particle-forming component to generate a particle-forming component-enclosed component-diluting solution mixed liquid, which may be supplied to the reaction chamber. In another embodiment, the particle-forming component and the enclosed component may first be mixed to generate a particle-forming component-enclosed component mixed liquid, and then the particle-forming component-enclosed component mixed liquid may be mixed with the diluting solution to generate a first-second-third mixed liquid, which may be supplied to the reaction chamber. In another embodiment, a particle-forming component solution may be first supplied from a first line 41A via the first pump 12 from the first tank 11 containing the particle-forming component solution, an enclosed component solution may be supplied from a second line 41B via the second pump 14 from the second tank 13 containing the enclosed component solution, and the solutions may be mixed to generate a particle-forming component-enclosed component mixed liquid in a mixed liquid line 41D, which may be supplied to the reaction chamber 5 via the first inlet 41. Then, the diluting solution may be supplied from the second inlet 42 via the third pump 16 to the reaction chamber 5 from a third tank 15 containing the diluting solution to generate a particle-forming component-enclosed component-diluting solution mixed liquid (see Figs. 1 and 5). The order of injecting the solution into the reaction chamber is not particularly limited. The diluting solution may be supplied to the reaction chamber first, and then the particle-forming component-enclosed component mixed liquid may be supplied to the reaction chamber. In addition, the diluting solution and the particle-forming component-enclosed component mixed liquid may be simultaneously supplied to the reaction chamber.

In a certain embodiment, the diluting solution may stabilize the enclosed component. In another embodiment, the diluting solution may reduce particle to particle association by diluting the formed capsule particle. The type of diluting solution may be any of a neutral buffer, an acidic buffer, and a basic buffer, and may be appropriately selected depending on the particle-forming component and the enclosed component. Examples of the diluting solution include, but are not limited to, citrate buffers, acetate buffers, phosphate buffers, and buffers containing Good's buffers. When the particle-forming component contains a pH-responsive cationic lipid and the enclosed component is a nucleic acid, the diluting solution can be preferably a neutral or weakly acidic buffer. For example, a phosphate buffer can be used as a neutral buffer, or a citrate buffer can be used as a weakly acidic buffer. When the particle-forming component includes a neutral amphiphilic lipid, the diluent may contain a near-neutral buffer, such as a phosphate buffer. When the particle-forming component includes an anionic lipid, the diluent may contain, for example, an alkaline or weakly alkaline buffer. In a certain embodiment, in the manufacture of the nucleic acid lipid particle, the same solvent as the solvent for the enclosed component (for example, if a citrate buffer is used as the solvent for the enclosed component, a citrate buffer) may be used as the diluting solution in order to reduce the content of the solvent in which the lipid is dissolved (e.g., ethanol) to stabilize the particle.

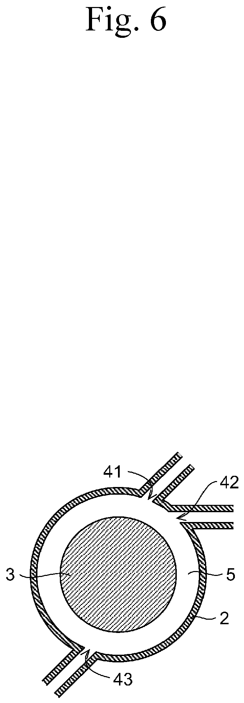

In another embodiment, the particle-forming component and the diluting solution may be first mixed to generate a particle-forming component-diluting solution mixed liquid, then the particle-forming component-diluting solution mixed liquid may be supplied to the reaction chamber, and then the enclosed component may be supplied to the reaction chamber to generate a particle-forming component-enclosed component-diluting solution mixed liquid. The order of injecting the solution into the reaction chamber is not particularly limited. The enclosed component may be supplied to the reaction chamber, and simultaneously or then the particle-forming component-diluting solution mixed liquid may be supplied to the reaction chamber. In another embodiment, the enclosed component and the diluting solution may be first mixed to generate an enclosed component-diluting solution mixed liquid, then the enclosed component-diluting solution mixed liquid may be supplied to the reaction chamber, and then the particle-forming component may be supplied to the reaction chamber to generate a particle-forming component-enclosed component-diluting solution mixed liquid. The order of injecting the solution into the reaction chamber is not particularly limited. The particle-forming component may be supplied to the reaction chamber, and simultaneously or then the enclosed component-diluting solution mixed liquid may be supplied to the reaction chamber. In another embodiment, the particle-forming component may be supplied to the reaction chamber 5 via the first inlet 41, the enclosed component may be supplied to the reaction chamber 5 via the second inlet 42, and the diluting solution may be supplied to the reaction chamber 5 via a third inlet 43 to generate a particle-forming component-enclosed component-diluting solution mixed liquid in the reaction chamber 5 (see Fig. 6). The order of injecting the solution into the reaction chamber is not particularly limited. The particle-forming component may be supplied to the reaction chamber, and simultaneously or then the diluting solution may be supplied to the reaction chamber, and simultaneously or then the enclosed component may be supplied to the reaction chamber. The enclosed component may be supplied to the reaction chamber, and simultaneously or then the particle-forming component may be supplied to the reaction chamber, and simultaneously or then the diluting solution may be supplied to the reaction chamber. The enclosed component may be supplied to the reaction chamber, and simultaneously or then the diluting solution may be supplied to the reaction chamber, and simultaneously or then the particle-forming component may be supplied to the reaction chamber. The diluting solution may be supplied to the reaction chamber, and simultaneously or then the particle-forming component may be supplied to the reaction chamber, and simultaneously or then the enclosed component may be supplied to the reaction chamber. The diluting solution may be supplied to the reaction chamber, simultaneously or then the enclosed component may be supplied to the reaction chamber, and simultaneously or then the particle-forming component may be supplied to the reaction chamber. The three liquids of the particle-forming component, the enclosed component, and the diluting solution may be simultaneously supplied to the reaction chamber.

The same applies to a solution other than the particle-forming component, the enclosed component, and/or the diluting solution. That is, in a certain embodiment, the particle manufacturing method of the present disclosure may further include a step of injecting one or more additional solutions in addition to the particle-forming component, the enclosed component, and/or the diluting solution. The additional solutions may each independently be a solution in which the particle-forming component is dissolved, a solution in which the enclosed component is dissolved, or a diluting solution, or a liquid for mixing these solutions.

In addition, with regard to the particle manufacturing method using the Taylor reactor 1 of the present disclosure, the particle is not necessarily manufactured only in the reaction chamber 5. For example, in an aspect in which the particle-forming component and the enclosed component are mixed and then the mixture is supplied to the reaction chamber 5 of the Taylor reactor 1, the target particles can be partially formed from the stage of mixing the particle-forming component and the enclosed component. The target particles formed by such a process also correspond to the target particles manufactured by the manufacturing method of the present disclosure or the target particles manufactured by the reactor of the present disclosure. In the case of the step of mixing in front of the reaction tank, not only the target particles are formed, but also the particles can be homogenized, in the reaction chamber 5.

In a certain embodiment, the Taylor reactor 1 has an inlet 4 (which is also the common inlet as claimed) for injecting a particle-forming component-enclosed component mixed liquid in which a particle-forming component and an enclosed component are mixed into a reaction chamber 5 (see Fig. 2). In a certain embodiment, the particle manufacturing method includes a step of injecting a particle-forming component-enclosed component mixed liquid from the inlet 4, rotating an inner cylinder 3, and further mixing the particle-forming component-enclosed component mixed liquid. In the reaction chamber 5, the particle-forming component-enclosed component mixed liquid may be subjected to a Taylor vortex flow reaction and further mixed.

In a certain embodiment, the Taylor reactor 1 has a first inlet 41 for injecting a particle-forming component into a reaction chamber 5 and a second inlet 42 for injecting an enclosed component into the reaction chamber 5 (see Fig. 3). In a certain embodiment, the particle manufacturing method includes a step of injecting a particle-forming component from the first inlet 41, injecting an enclosed component from the second inlet 42, rotating an inner cylinder 3, and mixing the particle-forming component and the enclosed component. In the reaction chamber 5, the particle-forming component and the enclosed component may be subjected to a Taylor vortex flow reaction.

In a certain embodiment, the Taylor reactor 1 may further have a third inlet 43 for injecting the diluting solution into the reaction chamber 5 (see Fig. 6). In a certain embodiment, the particle manufacturing method includes a step of injecting the diluting solution from the third inlet 43 and rotating the inner cylinder 3. In the reaction chamber 5, the diluting solution and the solution in the reaction chamber 5 may be subjected to a Taylor vortex flow reaction. The same applies to a fourth inlet for injecting a fourth solution, a fifth inlet for injecting a fifth solution, and an n-th inlet for injecting an n-th solution (n = a natural number of 6 or more). That is, in a certain embodiment, the Taylor reactor 1 of the present disclosure may further have a fourth inlet, a fifth inlet ... an n-th inlet.

In a certain embodiment, the particle manufactured may be a lipid particle. In a certain embodiment, the lipid particle may contain a lipid component and a component to be enclosed, such as a nucleic acid. In a certain embodiment, the particle-forming component may contain a lipid component, and the enclosed component may contain a component to be enclosed, such as a nucleic acid. In another embodiment, the particle-forming component may contain a component to be enclosed, such as a nucleic acid, and the enclosed component may contain a lipid component.

In a particular embodiment, the diluting solution may reduce a content of an organic solvent (such as ethanol) in which the lipid is dissolved and stabilize the particle. The diluting solution may be contacted with the solution containing the lipid component at any timing.

In a certain embodiment, the present disclosure provides a Taylor reactor 1 for manufacturing a particle. Examples of the particle to be manufactured include capsule particles, but are not limited thereto. In a certain embodiment, the Taylor reactor 1 has an inner cylinder 3 and an outer cylinder 2. A gap portion is formed between the outer diameter of the inner cylinder 3 and the inner diameter of the outer cylinder 2, and is used as the reaction chamber 5. Therefore, the Taylor reactor 1 includes the outer cylinder 2 and the inner cylinder 3, the inner cylinder 3 is rotatably disposed in the outer cylinder 2, and an annular reaction chamber 5 is formed between the inner cylinder 3 and the outer cylinder 2. The Taylor reactor 1 may have one or more inlets 4 for allowing the liquid to flow into the reaction chamber 5. The Taylor reactor 1 may have at least one outlet 6 for allowing the liquid in the reaction chamber 5 to flow out to the outside.

In a certain embodiment, the Taylor reactor 1 may be configured to mix the particle-forming component and the enclosed component "in front of the reaction chamber." In this case, the first line 4A from which the particle-forming component is injected and the second line 4B from which the enclosed component is injected merge (Fig. 2). A portion after a merging portion (that is, a downstream side) is sometimes referred to as particle-forming component-enclosed component mixed liquid line 4D or common line, for convenience, in the present specification. The particle-forming component and the enclosed component are mixed in the particle-forming component-enclosed component mixed liquid line 4D. The particle-forming component-enclosed component mixed liquid line 4D then supplies the particle-forming component-enclosed component mixed liquid into the reaction chamber 5 via the inlet 4 (which is also the common inlet as claimed). In another embodiment, the Taylor reactor 1 may be configured to mix the particle-forming component and the enclosed component "in the reaction chamber." In this case, the reaction chamber 5 is provided with a first inlet 41 (also referred to as first injection port) for injecting the particle-forming component and a second inlet 42 (also referred to as second injection port) for injecting the enclosed component (Fig. 3). In both the case of mixing the particle-forming component and the enclosed component "in front of the reaction chamber" and the case of mixing the particle-forming component and the enclosed component "in the reaction chamber", the order of injecting the solution is not particularly limited. The particle-forming component and the enclosed component may be injected simultaneously, the particle-forming component may be injected first, or the enclosed component may be injected first.

When the reaction chamber 5 is provided with the first inlet 41 for injecting the particle-forming component and the second inlet 42 for injecting the enclosed component, the first inlet 41 and the second inlet 42 can be disposed in any relative manner. In a certain embodiment, the first inlet 41 and the second inlet 42 may be disposed so as to form an angle of about 15 degrees, about 30 degrees, about 45 degrees, about 60 degrees, about 75 degrees, about 90 degrees, about 105 degrees, about 120 degrees, about 135 degrees, about 150 degrees, about 165 degrees, about 180 degrees, about 195 degrees, about 210 degrees, about 225 degrees, about 240 degrees, about 255 degrees, about 270 degrees, about 285 degrees, about 300 degrees, about 315 degrees, about 330 degrees, about 345 degrees, or about 360 degrees with respect to each other, as viewed from a direction of the rotation axis for rotating the inner cylinder 3 (as viewed from a direction perpendicular to a circular cross section of the inner cylinder 3), but the present invention is not limited thereto. Here, the "about" for the angle refers to a range of $\pm 7.5$ degrees of the indicated angle. In a certain embodiment, the first inlet 41 and the second inlet 42 may be adjacent to each other or may be separated from each other by a certain distance, as viewed from the direction perpendicular to the rotation axis for rotating the inner cylinder 3 (as viewed from a direction perpendicular to a rectangular cross section of the inner cylinder).

When the side on which the liquid is supplied to the reaction chamber 5 is defined as an upstream side and the side on which the liquid is discharged from the reaction chamber 5 is defined as a downstream side, the first inlet 41 may be disposed on the upstream side and the second inlet 42 may be disposed on the downstream side in a certain embodiment. In another embodiment, the second inlet 42 may be disposed on the upstream side, and the first inlet 41 may be disposed on the downstream side. In another embodiment, the first inlet 41 and the second inlet 42 may be disposed on the same cross section perpendicular to the axial direction of the inner cylinder 3 (Fig. 3). In this case, the first inlet 41 and the second inlet 42 are both on the same rotational cross section, not either on the upstream side or the downstream side. The third inlet 43 can also be arbitrarily disposed. That is, the third inlet 43 can be disposed on the upstream side of the first inlet 41, on the same rotational cross section as the first inlet 41, on the downstream side of the first inlet 41 and on the upstream side of the second inlet 42, on the same rotational cross section as the second inlet 42, or on the downstream side of the second inlet 42. Alternatively, the first inlet, second inlet, and third inlet 43 may be disposed on the same rotational cross section (Fig. 6). The same applies to the fourth inlet, the fifth inlet, ..., and the n-th inlet (n = a natural number of 6 or more).

In a particular embodiment, with regard to the particle manufacturing method and/or with regard to the Taylor reactor 1, a peripheral speed of the inner cylinder 3 can be, for example, 0.5 to 47 m/s, for example, 0.4 m/s or more, 0.41 m/s or more, 0.42 m/s or more, 0.43 m/s or more, 0.44 m/s or more, 0.45 m/s or more, 0.46 m/s or more, 0.47 m/s or more, 0.48 m/s or more, 0.49 m/s or more, 0.5 m/s or more, 0.51 m/s or more, 0.52 m/s or more, 0.53 m/s or more, 0.54 m/s or more, 0.55 m/s or more, 0.6 m/s or more, 0.65 m/s or more, 0.7 m/s or more, 0.75 m/s or more, 0.8 m/s or more, 0.85 m/s or more, 0.9 m/s or more, 0.95 m/s or more, 1 m/s or more, 1.5 m/s or more, 2 m/s or more, 2.5 m/s or more, 3 m/s or more, 4 m/s or more, 5 m/s or more, 6 m/s or more, 7 m/s or more, 8 m/s or more, 9 m/s or more, 10 m/s or more, 11 m/s or more, 12 m/s or more, 13 m/s or more, 14 m/s or more, 15 m/s or more, 20 m/s or more, 25 m/s or more, 30 m/s or more, 35 m/s or more, 40 m/s or more, 41 m/s or more, 42 m/s or more, 43 m/s or more, 44 m/s or more, 45 m/s or more, 46 m/s or more, for example, 47 m/s or more, and, for example, 55 m/s or less, 54 m/s or less, 53 m/s or less, 52 m/s or less, 51 m/s or less, 50 m/s or less, 49 m/s or less, 48 m/s or less, 47 m/s or less, 46 m/s or less, 45 m/s or less, 44 m/s or less, 43 m/s or less, 42 m/s or less, 41 m/s or less, 40 m/s or less, 35 m/s or less, 30 m/s or less, 25 m/s or less, 20 m/s or less, 15 m/s or less, 14 m/s or less, 13 m/s or less, 12 m/s or less, 11 m/s or less, 10 m/s or less, 9 m/s or less, 8 m/s or less, 7 m/s or less, 6 m/s or less, 5 m/s or less, 4 m/s or less, 3 m/s or less, 2 m/s or less, 1 m/s or less, 0.95 m/s or less, 0.9 m/s or less, 0.85 m/s or less, 0.8 m/s or less, 0.75 m/s or less, 0.7 m/s or less, 0.65 m/s or less, 0.6 m/s or less, 0.55 m/s or less, 0.54 m/s or less, 0.53 m/s or less, 0.52 m/s or less, 0.51 m/s or less, 0.5 m/s or less, 0.49 m/s or less, 0.48 m/s or less, 0.47 m/s or less, 0.46 m/s or less, 0.45 m/s or less, 0.44 m/s or less, 0.43 m/s or less, 0.42 m/s or less, for example, 0.41 m/s or less, for example, any combination of these upper and lower limits, for example, 0.4 to 55 m/s, 0.45 to 54 m/s, 0.5 to 53 m/s, 0.5 to 52 m/s, 0.5 to 51 m/s, 0.5 to 50 m/s, 0.5 to 49 m/s, 0.5 to 48 m/s, 0.5 to 47 m/s, 0.51 to 46 m/s, 0.52 to 45 m/s, 0.53 to 44 m/s, 0.54 to 43 m/s, 0.55 to 42 m/s, 0.6 to 41 m/s, 0.7 to 40 m/s, 0.8 to 35 m/s, 0.9 to 30 m/s, 1 to 25 m/s, 1.5 to 20 m/s, 2 to 15 m/s, 3 to 14 m/s, 4 to 13 m/s, 5 to 12 m/s, 6 to 11 m/s, 7 to 10 m/s, for example, 8 to 9 m/s, but is not limited thereto. In the present specification, unless otherwise specified, the peripheral speed is defined as:

peripheral speed = outer diameter of inner cylinder $\times \pi \times$ rotation speed          [Mathematical formula 2]

(wherein $\pi$ represents a circular constant).

In a particular embodiment, when the inner diameter of the outer cylinder is $\varphi$ 30 mm, the rotation speed of the inner cylinder 3 can be, for example, but not limited to, 500 to 30000 rpm, 550 to 25000 rpm, 600 to 20000 rpm, 650 to 15000 rpm, 700 to 10000 rpm, 750 to 9000 rpm, 800 to 8000 rpm, 850 to 7000 rpm, 900 to 6000 rpm, for example, 1000 to 6000 rpm. In a particular embodiment, when the inner diameter of the outer cylinder is $\varphi$ 30 mm, the outer diameter of the inner cylinder can be $\varphi$ 20 mm or more, $\varphi$ 21 mm or more, $\varphi$ 22 mm or more, $\varphi$ 23 mm or more, $\varphi$ 24 mm or more, $\varphi$ 25 mm or more, $\varphi$ 26 mm or more, $\varphi$ 27 mm or more, $\varphi$ 28 mm or more, $\varphi$ 29 mm or more, $\varphi$ 29.1 mm or more, $\varphi$ 29.2 mm or more, $\varphi$ 29.3 mm or more, $\varphi$ 29.4 mm or more, $\varphi$ 29.5 mm or more, $\varphi$ 29.6 mm or more, $\varphi$ 29.7 mm or more, $\varphi$ 29.8 mm or more, $\varphi$ 29.9 mm or more, $\varphi$ 29.91 mm or more, $\varphi$ 29.92 mm or more, $\varphi$ 29.93 mm or more, $\varphi$ 29.94 mm or more, $\varphi$ 29.95 mm or more, $\varphi$ 29.96 mm or more, $\varphi$ 29.97 mm or more, $\varphi$ 29.98 mm or more, or $\varphi$ 29.99 mm or more, but is not limited thereto.

In a particular embodiment, with regard to the particle manufacturing method and/or with regard to the Taylor reactor 1, a gap width d between the inner cylinder 3 and the outer cylinder 2 can be 0.01 mm or more, 0.02 mm or more, 0.03 mm or more, 0.04 mm or more, 0.05 mm or more, 0.06 mm or more, 0.07 mm or more, 0.08 mm or more, 0.09 mm or more, 0.1 mm or more, 0.11 mm or more, 0.12 mm or more, 0.13 mm or more, 0.14 mm or more, 0.15 mm or more, for example, 0.21 mm or more, 0.75 mm or more, 1.5 mm or more, and, for example, 5 mm or less, 4.9 mm or less, 4.8 mm or less, 4.7 mm or less, 4.6 mm or less, 4.5 mm or less, 4.4 mm or less, 4.3 mm or less, 4.2 mm or less, 4.1 mm or less, 4 mm or less, 3.5 mm or less, 3 mm or less, 2.5 mm or less, 2 mm, 1.5 mm or less, 1.0 mm or less, for example, 0.01 mm to 5 mm, 0.02 mm to 4.9 mm, 0.03 mm to 4.8 mm, 0.04 mm to 4.7 mm, 0.05 mm to 4.6 mm, 0.06 mm to 4.5 mm, 0.07 mm to 4.4 mm, 0.08 mm to 4.3 mm, 0.09 mm to 4.2 mm, 0.1 mm to 4.1 mm, 0.11 mm to 4 mm, 0.12 mm to 3.5 mm, 0.13 mm to 3 mm, 0.14 mm to 2 mm, for example, 0.15 mm to 1.5 mm, but is not limited thereto.

With regard to the particle manufacturing method and/or with regard to the Taylor reactor 1, the peripheral speed and the gap width d can be set so as to obtain a stirring force R as a formula in which a shearing force is simplified. The stirring force R is calculated by the following formula.

[Mathematical formula 3]

$$\text{Stirring force } R = \text{peripheral speed} \div \text{gap width } d$$

In a certain embodiment, the rotation speed of the inner cylinder 3 and the gap width d between the inner cylinder 3 and the outer cylinder 2 may be set so that the stirring force R is in a range of $0.5/5 < R < 47/0.01$, 100 to 5, 000, 000 s$^{-1}$, 104 to 4,900,000 s$^{-1}$, 105 to 4,900,000 s$^{-1}$, 110 to 4,800,000 s$^{-1}$, 120 to 4,710,000 s$^{-1}$, 130 to 4,700,900 s$^{-1}$, 140 to 4,700,000 s$^{-1}$, 150 to 4,600,000 s$^{-1}$, 160 to 4,500,000 s$^{-1}$, 170 to 4,400,000 s$^{-1}$, 180 to 4,300,000 s$^{-1}$, 190 to 4,200,000 s$^{-1}$, 200 to 4,100,000 s$^{-1}$, 250 to 4,000,000 s$^{-1}$, 300 to 3,900,000 s$^{-1}$, 400 to 3,800,000 s$^{-1}$, 500 to 3,700,000 s$^{-1}$, 600 to 3,600,000 s$^{-1}$, 700 to 3,500,000 s$^{-1}$, 800 to 3,400,000 s$^{-1}$, 900 to 3,300,000 s$^{-1}$, 1000 to 3,200,000 s$^{-1}$, 1500 to 3,100,000 s$^{-1}$, 2000 to 3,000,000 s$^{-1}$, 3000 to 2,500,000 s$^{-1}$, 4000 to 2,000,000 s$^{-1}$, 5000 to 1,500,000 s$^{-1}$, 10,000 to 1,000,000 s$^{-1}$, or any combination of these upper and lower limits.

The solutions can be mixed with each other and injected into the reaction chamber 5 at an appropriate injection rate. Those skilled in the art can appropriately set the injection rate of the solution. Those skilled in the art can appropriately set a ratio between the injection rates of the solutions. In a certain embodiment, a ratio of the injection rate of the particle-forming component to the injection rate of the enclosed component can be, but is not limited to, a ratio selected from a range of 1:1 to 1:9, such as 1:2 to 1:9, 1:3 to 1:9, such as 1:1, 1:2, 1:3, 1:4, 1:5, 1:6, 1:7, 1:8, such as 1:9. The injection rate may be determined depending on the combination of the particle-forming component and the enclosed component. The injection rate of the particle-forming component or the enclosed component may be, for example, 1 mL/min or more, 1.1 mL/min or more, 1.2 mL/min or more, 1.3 mL/min or more, 1.4 mL/min or more, 1.5 mL/min or more, 1.6 mL/min or more, 1.7 mL/min or more, 1.8 mL/min or more, 1.9 mL/min or more, 2 mL/min or more, 2.1 mL/min or more, 2.2 mL/min or more, 2.3 mL/min or more, 2.4 mL/min or more, 2.5 mL/min or more, 2.6 mL/min or more, 2.7 mL/min or more, 2.8 mL/min or more, 2.9 mL/min or more, 3 mL/min or more, 3.5 mL/min or more, 4 mL/min or more, 4.5 mL/min or more, 5 mL/min or more, 6 mL/min or more, 7 mL/min or more, 8 mL/min or more, 9 mL/min or more, 10 mL/min or more, 20 mL/min or more, 30 mL/min or more, 40 mL/min or more, 50 mL/min or more, 60 mL/min or more, 70 mL/min or more, 80 mL/min or more, 90 mL/min or more, 100 mL/min or more, 110 mL/min or more, 120 mL/min or more, 130 mL/min or more, 140 mL/min or more, such as 150 mL/min or more, and 150 mL/min or less, 140 mL/min or less, 130 mL/min or less, 120 mL/min or less, 110 mL/min or less, 100 mL/min or less, 90 mL/min or less, 80 mL/min or less, 70 mL/min or less, 60 mL/min or less, 50 mL/min or less, 40 mL/min or less, 30 mL/min or less, 20 mL/min or less, 10 mL/min or less, 9 mL/min or less, 8 mL/min or less, 7 mL/min or less, 6 mL/min or less, 5 mL/min or less, 4.5 mL/min or less, 4 mL/min or less, 3.5 mL/min or less, 3 mL/min or less, 2.9 mL/min or less, 2.8 mL/min or less, 2.7 mL/min or less, 2.6 mL/min or less, 2.5 mL/min or less, 2.4 mL/min or less, 2.3 mL/min or less, 2.2 mL/min or less, 2.1 mL/min or less, 2 mL/min or less, 1.9 mL/min or less, 1.8 mL/min or less, 1.7 mL/min or less, 1.6 mL/min or less, 1.5 mL/min or less, 1.4 mL/min or less, 1.3 mL/min or less, 1.2 mL/min or less, 1.1 mL/min or less, such as 1 mL/min or less.

In a certain embodiment, a stirring time for performing a Taylor reaction may be 5 minutes or less, 4 minutes or less, 3 minutes or less, 2 minutes or less, 90 seconds or less, 60 seconds or less, 50 seconds or less, 40 seconds or less, 30 seconds or less, 20 seconds or less, 15 seconds or less, for example, 10 seconds or less, but is not limited thereto. In a certain embodiment, the stirring time for performing a Taylor reaction may be 1 second or more, 2 seconds or more, 3 seconds or more, 4 seconds or more, for example, 5 seconds or more, but is not limited thereto. In the present specification, the "stirring time" refers to a time from when an injected liquid flows into the reaction tank to when the liquid passes through an outlet port, unless otherwise specified. The time from when the injected liquid flows into the reaction tank to when the liquid passes through the outlet port is sometimes referred to as time for one pass. In addition, in a case where there are multiple inlets, the stirring time refers to a time from when the slowest injected liquid flows into the reaction tank to when the liquid passes through the outlet port.

In a certain embodiment, inner diameters of the first inlet 41 and the second inlet 42 may be the same or substantially the same. The term "substantially the same" includes ±5%. In another embodiment, the inner diameters of the first inlet 41 and the second inlet 42 may be different.

EXAMPLES

Hereinafter, the present invention will be specifically described by way of examples. These examples are intended to illustrate the present invention, and do not limit the scope of the present invention.

[Example 1]

Preparation of siRNA-enclosed nucleic acid lipid particles

As specimens 1 to 3, siRNA-enclosed nucleic acid lipid particles were prepared as follows. Distearoylphosphatidylcholine (1,2-Distearoyl-sn-glycero-3-phosphocholine: hereinafter referred to as DSPC, NOF CORPORATION), cholesterol (Cholesterol: hereinafter referred to as Chol, Sigma-Aldrich, Inc.), a cationic lipid 1 (compound described in WO 2015/005253) (hereinafter referred to as LP1), and 1,2-dimyristoyl-sn-glycerol methoxypolyethyleneglycol (1,2-Dimyristoyl-sn-Glycero-3-Methoxypolyethyleneglycol, hereinafter referred to as PEG2000-DMG, NOF CORPORATION) having a polyethylene glycol molecular weight of about 2000 were dissolved in ethanol at a molar ratio of DSPC:Chol:LP1:PEG2000-DMG = 10:48:40:2 so as to attain a total lipid concentration of 1.96 mg/mL.
On the other hand, siRNA for Transferin Receptor 2 (AD-47882) was dissolved in a citrate buffer (20 mM Citrate Buffer, pH 4.0) to attain 51.4 $\mu$g/mL. AD-47882 is siRNA for mouse TfR2 described in WO 2012/177921.
The lipid solution and the siRNA solution were mixed by injecting the lipid solution and the siRNA solution from "injection ports" for the respective solutions shown in Table 1 into a Taylor reactor in which the inner cylinder rotated at a "peripheral speed" shown in Table 1 and whish has a "gap width" between the inner cylinder and the outer cylinder shown in Table 1, by using a pump at an "injection rate (sum of injection rates of the lipid solution and the siRNA solution)" shown in Table 1, whereby a dispersion of nucleic acid lipid particles was obtained from an outlet port. The injection rate ratio between the lipid solution and the siRNA solution was 1:3. In the reactor of this example, the first inlet was on the upstream side, the second inlet was on the downstream side, the siRNA solution as an enclosed component was injected from the first inlet, and the lipid solution as a particle-forming component was injected from the second inlet. Regarding "Injection port" shown in Table 1, "In reaction tank (in reaction chamber)" and "In front of reaction tank (in front of reaction chamber)" are conditions related to the mixing position of the two liquids. When the injection port is "In reaction tank", the two liquids can be mixed in the reaction tank. If the injection port is "In front of reaction tank", the two liquids can be mixed in front of the reaction tank, and then the mixed liquid can be injected into a reaction section of the Taylor reactor, that is, a gap portion between the inner cylinder and the outer cylinder. The same applies to Tables 2 to 5. Regarding "Diluted or not" shown in Table 1, in the case of diluting the nucleic acid lipid particles, a citrate buffer (20 mM Citrate Buffer, pH 4.0) was injected as a diluting solution from the injection port before the outlet port at the equal rate to the "Injection rate" shown in Table 1 using a pump. The obtained dispersion of nucleic acid lipid particles was dialyzed (Float-A-Lyzer G2, MWCO: 1,000 kD, Spectra/Por) against about 25 to 50 times the amount of PBS (pH 7.4) for 12 to 18 hours to remove ethanol, and a purified dispersion of siRNA-enclosed nucleic acid lipid particles was obtained.
LP1 was synthesized according to the method described in WO 2015/005253. The description of LP1 synthesis in WO 2015/005253 is incorporated herein by reference.

[Example 2]

Preparation of dsDNA-enclosed nucleic acid lipid particles

As specimens 4 to 8, dsDNA-enclosed nucleic acid lipid particles were prepared as follows. Distearoylphosphatidylcholine (1,2-Distearoyl-sn-glycero-3-phosphocholine: hereinafter referred to as DSPC, NOF CORPORATION), cholesterol (Cholesterol: hereinafter referred to as Chol, Sigma-Aldrich, Inc.), LP1, and 1,2-dimyristoyl-sn-glycerol methoxypolyethyleneglycol (1,2-Dimyristoyl-sn-Glycero-3-Methoxypolyethyleneglycol, hereinafter referred to as PEG2000-DMG, NOF CORPORATION) having a polyethylene glycol molecular weight of about 2000 were dissolved in ethanol at a molar ratio of DSPC:Chol:LP1:PEG2000-DMG = 12.5:41:45:1.5 so as to attain a total lipid concentration of 3.16 mg/mL.
On the other hand, $\beta$-catenin dsDNA was prepared by dilution with a citrate buffer (20 mM Citrate Buffer, pH 4.0) to attain 52.7 $\mu$g/mL.
The lipid solution and the dsDNA solution were mixed by injecting the lipid solution and the dsDNA solution from "injection ports" shown in Table 2 into a Taylor reactor in which the inner cylinder rotated at a "peripheral speed" shown in Table 2 and which has a "gap width" between the inner cylinder and the outer cylinder shown in Table 2, by using a pump at an "injection rate (sum of injection rates of the lipid solution and the dsDNA solution)" shown in Table 2, whereby a dispersion of nucleic acid lipid particles was obtained from an outlet port. The injection rate ratio between the lipid solution and the dsDNA solution was 1:3. Regarding "Diluted or not" shown in Table 2, in the case of diluting the nucleic acid lipid particles, a citrate buffer (20 mM Citrate Buffer, pH 4.0) was injected as a diluting solution from the injection port before the outlet port at the equal rate to the "injection rate" shown in Table 2 using a pump. The dispersion of nucleic acid lipid particles was dialyzed (Float-A-Lyzer G2, MWCO: 1,000 kD, Spectra/Por) against about 25 to 50 times the amount of PBS (pH 7.4) for 12 to 18 hours to remove ethanol, and a purified dispersion of dsDNA-enclosed nucleic acid lipid particles was obtained.

LP1 was synthesized according to the method described in WO 2015/005253.

[Example 3]

Preparation of mRNA-enclosed nucleic acid lipid particles

As specimens 9 to 11, mRNA-enclosed nucleic acid lipid particles were prepared as follows. Distearoylphosphatidylcholine (1,2-Distearoyl-sn-glycero-3-phosphocholine: hereinafter referred to as DSPC, NOF CORPORATION), cholesterol (Cholesterol: hereinafter referred to as Chol, Sigma-Aldrich, Inc.), LP1, and 1,2-dimyristoyl-sn-glycerol methoxypolyethyleneglycol (1,2-Dimyristoyl-sn-Glycero-3-Methoxypolyethyleneglycol, hereinafter referred to as PEG2000-DMG, NOF CORPORATION) having a polyethylene glycol molecular weight of about 2000 were dissolved in ethanol at a molar ratio of DSPC:Chol:LP1:PEG2000-DMG = 12.5:41:45:1.5 so as to attain a total lipid concentration of 3.16 mg/mL.

On the other hand, A_Sin _GP1908_2015_H1 mRNA was prepared by dilution with a citrate buffer (20 mM Citrate Buffer, pH 4.0) to attain 52.7 $\mu$g/mL.

The lipid solution and the mRNA solution were mixed by injecting the lipid solution and the mRNA solution from "injection ports" shown in Table 3 into a Taylor reactor in which the inner cylinder rotated at a "peripheral speed" shown in Table 3 and which has a "gap width" between the inner cylinder and the outer cylinder shown in Table 3, by using a pump at an "injection rate (sum of injection rates of the lipid solution and the mRNA solution)" shown in Table 3, whereby a dispersion of nucleic acid lipid particles was obtained from an outlet port. The injection rate ratio between the lipid solution and the mRNA solution was 1:3. Regarding "Diluted or not" shown in Table 3, in the case of diluting the nucleic acid lipid particles, a citrate buffer (20 mM Citrate Buffer, pH 4.0) was injected as a diluting solution from the injection port before the outlet port at the equal rate to the "injection rate" shown in Table 3 using a pump. The dispersion of nucleic acid lipid particles was dialyzed (Float-A-Lyzer G2, MWCO: 1,000 kD, Spectra/Por) against about 25 to 50 times the amount of PBS (pH 7.4) for 12 to 18 hours to remove ethanol, and a purified dispersion of mRNA-enclosed nucleic acid lipid particles was obtained.

LP1 was synthesized according to the method described in WO 2015/005253.

[Example 4]

Preparation of mRNA-enclosed nucleic acid lipid particles

As specimens 12 and 13, mRNA-enclosed nucleic acid lipid particles were prepared as follows. Distearoylphosphatidylcholine (1,2-Distearoyl-sn-glycero-3-phosphocholine: hereinafter referred to as DSPC, NOF CORPORATION), cholesterol (Cholesterol: hereinafter referred to as Chol, Sigma-Aldrich, Inc.), LP1, and 1,2-dimyristoyl-sn-glycerol methoxypolyethyleneglycol (1,2-Dimyristoyl-sn-Glycero-3-Methoxypolyethyleneglycol, hereinafter referred to as PEG2000-DMG, NOF CORPORATION) having a polyethylene glycol molecular weight of about 2000 were dissolved in ethanol at a molar ratio of DSPC:Chol:LP1:PEG2000-DMG = 12.5:41:45:1.5 so as to attain a total lipid concentration of 3.16 mg/mL.

On the other hand, A_Sin _GP1908_2015_H1 mRNA was prepared by dilution with a citrate buffer (20 mM Citrate Buffer, pH 4.0) to attain 52.7 $\mu$g/mL.

The lipid solution and the mRNA solution were mixed by injecting the lipid solution and the mRNA solution from "injection ports" shown in Table 4 into a Taylor reactor in which the inner cylinder rotated at a "peripheral speed" shown in Table 4 and which has a "gap width" between the inner cylinder and the outer cylinder shown in Table 4, by using a pump at an "injection rate (sum of injection rates of the lipid solution and the mRNA solution)" shown in Table 4, whereby a dispersion of nucleic acid lipid particles was obtained from an outlet port. The injection rate ratio between the lipid solution and the mRNA solution was 1:3. A citrate buffer (20 mM Citrate Buffer, pH 4.0) was injected as a diluting solution from the injection port before the outlet port at the equal rate to the "injection rate" shown in Table 4 using a pump. The dispersion of nucleic acid lipid particles was dialyzed (Float-A-Lyzer G2, MWCO: 1,000 kD, Spectra/Por) against about 25 to 50 times the amount of 10 mM Histidine 300 mM Sucrose (pH 7.0) for 12 to 18 hours to remove ethanol, and a purified dispersion of mRNA-enclosed nucleic acid lipid particles was obtained.

LP1 was synthesized according to the method described in WO 2015/005253.

[Example 5]

Preparation of mRNA-enclosed nucleic acid lipid particles

As a specimen 14, mRNA-enclosed nucleic acid lipid particles were prepared as follows. Distearoylphosphatidylcholine (1,2-Distearoyl-sn-glycero-3-phosphocholine: hereinafter referred to as DSPC, NOF CORPORATION), cholesterol (Cholesterol: hereinafter referred to as Chol, Sigma-Aldrich, Inc.), a cationic lipid 2 (compound described in WO 2015/005253) (hereinafter referred to as LP2), and 1,2-dimyristoyl-sn-glycerol methoxypolyethyleneglycol (1,2-Dimyristoyl-sn-Glycero-3-Methoxypolyethyleneglycol, hereinafter referred to as PEG2000-DMG, NOF CORPORATION) having a polyethylene glycol molecular weight of about 2000 were dissolved in ethanol at a molar ratio of DSPC:Chol:LP2:PEG2000-DMG = 17.5:21:60:1.5 so as to attain a total lipid concentration of 3.08 mg/mL.
On the other hand, A_Sin _GP1908_2015_H1 mRNA was prepared by dilution with a citrate buffer (20 mM Citrate Buffer, pH 4.0) to attain 51.3 $\mu$g/mL.
The lipid solution and the mRNA solution were mixed by injecting the lipid solution and the mRNA solution from "injection ports" shown in Table 5 into a Taylor reactor in which the inner cylinder rotated at a "peripheral speed" shown in Table 5 and which has a "gap width" between the inner cylinder and the outer cylinder shown in Table 5, by using a pump at an "injection rate (sum of injection rates of the lipid solution and the mRNA solution)" shown in Table 5, and a dispersion of nucleic acid lipid particles was obtained from an outlet port. The injection rate ratio between the lipid solution and the mRNA solution was 1:3. A citrate buffer (20 mM Citrate Buffer, pH 4.0) was injected as a diluting solution from the injection port before the outlet port at the equal rate to the "injection rate" shown in Table 5 using a pump. The dispersion of nucleic acid lipid particles was dialyzed (Float-A-Lyzer G2, MWCO: 1,000 kD, Spectra/Por) against about 25 to 50 times the amount of 10 mM Histidine 300 mM Sucrose (pH 7.0) for 12 to 18 hours to remove ethanol, and a purified dispersion of mRNA-enclosed nucleic acid lipid particles was obtained.
LP2 was synthesized according to the method described in WO 2015/005253. The description of LP2 synthesis in WO 2015/005253 is incorporated herein by reference.

[Example 6]

Characterization of nucleic acid-enclosed nucleic acid lipid particles

For the specimens 1 to 14, characterization of the dispersions containing nucleic acid lipid particles was performed. Characterization methods will be described.

(1) Nucleic acid enclosure rate

The nucleic acid enclosure rate was measured according to the package insert using Quant-iT RiboGreen RNA Assay Kit (Invitrogen).
That is, the nucleic acid in the dispersion of nucleic acid lipid particles was quantified in the presence and absence of 0.015% Triton X-100 surfactant, and the enclosure rate was calculated by the following formula.

{([Amount of nucleic acid in presence of surfactant] - [Amount of nucleic acid in absence of surfactant])/[Amount of nucleic acid in presence of surfactant]} $\times$ 100 (%)      [Mathematical Formula 4]

(2) Mean particle diameter/polydispersity index (PDI)

The mean particle diameter and polydispersity index of the nucleic acid lipid particles were measured with Zeta Potential/Particle Sizer NICOMP (trademark) 380ZLS (PARTICLE SIZING SYSTEMS). The mean particle diameter in the table represents a mean volume diameter. In addition, the PDI is a polydispersity index calculated by the square of (standard deviation/mean particle diameter), and is sometimes simply referred to as degree of dispersion.
The results of the evaluation of the characteristics are shown in Tables 1 to 5. As shown in the tables, nucleic acid lipid particles having a mean particle diameter of 80 to 200 nm and a nucleic acid enclosure rate of 85% or more were obtained. This demonstrates that a Taylor reactor can manufacture lipid particles having nucleic acids enclosed therein. In addition, it has been demonstrated that particles that can be manufactured by a microchannel can also be manufactured by a Taylor reactor.

(Reference Examples) Preparation of nucleic acid lipid particles by microchannel mixing

(Reference Example 1) Preparation and characterization of siRNA-enclosed nucleic acid lipid particles

Reference Example 1 was prepared as follows. DSPC, Chol, LP1 and PEG2000-DMG were dissolved in ethanol at a molar ratio of DSPC:Chol:LP1:PEG2000-DMG = 10:48:40:2 so as to attain a total lipid concentration of 1.96 mg/mL. On the other hand, siRNA for Transferin Receptor 2 (AD-47882) was dissolved in a citrate buffer (20 mM Citrate Buffer, pH 4.0) to attain 51.4 $\mu$g/mL. The lipid solution and the mRNA solution were mixed in a microchannel using NanoAssemblr BenchTop (Precision Nanosystems Inc.) at a volume ratio of 1:3 to obtain a crude dispersion of nucleic acid lipid particles. The dispersion of nucleic acid lipid particles was dialyzed (Float-A-Lyzer G2, MWCO: 1,000 kD, Spectra/Por) against about 25 to 50 times the amount of a buffer for 12 to 18 hours to remove ethanol, and a purified dispersion of siRNA-enclosed nucleic acid lipid particles was obtained.

The obtained nucleic acid lipid particles of Reference Example 1 were characterized in the same manner as in Example 6, and the results are shown in Table 6.

(Reference Examples 2 and 3) Preparation and characterization of mRNA-enclosed nucleic acid lipid particles

Reference Examples 2 and 3 were prepared as follows. DSPC, Chol, LP1 and PEG2000-DMG were dissolved in ethanol at a molar ratio of DSPC:Chol:LP1:PEG2000-DMG = 12.5:41:45:1.5 so as to attain a total lipid concentration of 3.16 mg/mL. On the other hand, A_Sin_GP1908_2015_H1 mRNA (sequence information) was prepared by dilution with a citrate buffer (20 mM Citrate Buffer, pH 4.0) to attain 52.7 $\mu$g/mL. The lipid solution and the mRNA solution were mixed in a microchannel using NanoAssemblr BenchTop (Precision Nanosystems Inc.) at a volume ratio of 1:3 to obtain a crude dispersion of nucleic acid lipid particles. The dispersion of nucleic acid lipid particles was dialyzed (Float-A-Lyzer G2, MWCO: 1,000 kD, Spectra/Por) against about 25 to 50 times the amount of a buffer for 12 to 18 hours to remove ethanol, and a purified dispersion of mRNA-enclosed nucleic acid lipid particles was obtained.

The obtained nucleic acid lipid particles of Reference Examples 2 and 3 were characterized in the same manner as in Example 6, and the results are shown in Table 6.

(Reference Example 4) Preparation and characterization of mRNA-enclosed nucleic acid lipid particles

Reference Example 4 was prepared as follows. DSPC, Chol, LP2 and PEG2000-DMG were dissolved in ethanol at a molar ratio of DSPC:Chol:LP2:PEG2000-DMG = 17.5:21:60:1.5 so as to attain a total lipid concentration of 3.08 mg/mL. On the other hand, A_Sin_GP1908_2015_H1 mRNA (sequence information) was prepared by dilution with a citrate buffer (20 mM Citrate Buffer, pH 4.0) to attain 51.3 $\mu$g/mL. The lipid solution and the mRNA solution were mixed in a microchannel using NanoAssemblr BenchTop (Precision Nanosystems Inc.) at a volume ratio of 1:3 to obtain a crude dispersion of nucleic acid lipid particles. The dispersion of nucleic acid lipid particles was dialyzed (Float-A-Lyzer G2, MWCO: 1,000 kD, Spectra/Por) against about 25 to 50 times the amount of a buffer for 12 to 18 hours to remove ethanol, and a purified dispersion of mRNA-enclosed nucleic acid lipid particles was obtained.

The obtained nucleic acid lipid particles of Reference Example 4 were characterized in the same manner as in Example 6, and the results are shown in Table 6.

As shown in Table 6, for References Examples 1 to 4, nucleic acid lipid particles having a mean particle diameter of 90 to 140 nm and a nucleic acid enclosure rate of 95% or more were obtained.

[Table 1]

| Specimen | Injection port | Gap width (mm) | Peripheral speed (m/s) | Reynolds number | Stirring time (sec) | Injection rate (mL/min) | Diluted or not | Mean particle diameter (nm) | PDI | Nucleic acid enclosure rate (%) |
|---|---|---|---|---|---|---|---|---|---|---|
| 1 | In reaction tank | 0.21 | 9.29 | 770 | 3 | 32 | Not diluted | 98 | 0.043 | 97 |
| 2 | In reaction tank | 0.21 | 0.77 | 64 | 20 | 2.4 | Diluted | 129 | 0.024 | 98 |
| 3 | In reaction tank | 0.75 | 8.95 | 2649 | 60 | 2.8 | Diluted | 128 | 0.105 | 97 |
| TfR2 siRNA DSPC:Chol:LP1:PEG2000-DMG = 10:48:40:2 | | | | | | | | | | |

[Table 2]

| Specimen | Injection port | Gap width (mm) | Peripheral speed (m/s) | Reynolds number | Stirring time (sec) | Injection rate (mL/min) | Diluted or not | Mean particle diameter (nm) | PDI | Nucleic acid enclosure rate (%) |
|---|---|---|---|---|---|---|---|---|---|---|
| 4 | In front of reaction tank | 1.5 | 8.48 | 5021 | 2.5 | 128 | Not diluted | 145 | 0.269 | 93 |
| 5 | In front of reaction tank | 0.21 | 0.77 | 64 | 10 | 4.8 | Not diluted | 182 | 0.075 | 98 |
| 6 | In front of reaction tank | 0.21 | 9.29 | 770 | 0.5 | 96 | Diluted | 101 | 0.030 | 96 |
| 7 | In reaction tank | 0.21 | 9.29 | 770 | 10 | 4.8 | Diluted | 116 | 0.112 | 94 |
| 8 | In reaction tank | 0.21 | 4.65 | 385 | 5 | 9.6 | Diluted | 83 | 0.181 | 95 |

β catenin dsDNA
DSPC:Chol:LP1:PEG2000-DMG = 12.5:41:45:1.5

[Table 3]

| Specimen | Injection port | Gap width (mm) | Peripheral speed (m/s) | Reynolds number | Stirring time (sec) | Injection rate (mL/min) | Diluted or not | Mean particle diameter (nm) | PDI | Nucleic acid enclosure rate (%) |
|---|---|---|---|---|---|---|---|---|---|---|
| 9 | In reaction tank | 0.21 | 0.77 | 64 | 3 | 32 | Not diluted | 161 | 0.012 | 97 |
| 10 | In reaction tank | 0.21 | 9.29 | 770 | 20 | 2.4 | Diluted | 140 | 0.104 | 94 |
| 11 | In reaction tank | 0.75 | 8.95 | 2649 | 60 | 2.8 | Diluted | 151 | 0.028 | 98 |

A_Sin _GP1908_2015_H1 mRNA
DSPC:Chol:LP1:PEG2000-DMG = 12.5:41:45:1.5

[Table 4]

| Specimen | Injection port | Gap width (mm) | Peripheral speed (m/s) | Reynolds number | Stirring time (sec) | Injection rate (mL/min) | Diluted or not | Mean particle diameter (nm) | PDI | Nucleic acid enclosure rate (%) |
|---|---|---|---|---|---|---|---|---|---|---|
| 12 | In reaction tank | 0.21 | 9.29 | 770 | 1 | 48 * | Diluted | 98 | 0.036 | 97 |
| 13 | In reaction tank | 1.5 | 8.48 | 5021 | 4 | 81 * | Diluted | 89 | 0.074 | 97 |

*Syringe pump
A_Sin _GP1908_2015_H1 mRNA
DSPC:Chol:LP2:PEG2000-DMG = 12.5:41:45:1.5

[Table 5]

| Specimen | Injection port | Gap width (mm) | Peripheral speed (m/s) | Reynolds number | Stirring time (sec) | Injection rate (mL/min) | Diluted or not | Mean particle diameter (nm) | PDI | Nucleic acid enclosure rate (%) |
|---|---|---|---|---|---|---|---|---|---|---|
| 14 | In reaction tank | 0.21 | 9.29 | 770 | 1 | 48 * | Diluted | 137 | 0.083 | 89 |

*Syringe pump
A_Sin _GP1908_2015_H1 mRNA
DSPC:Chol:LP2:PEG2000-DMG = 17.5:21:60:1.5

[Table 6]

| Reference Example | Nucleic acid | Lipid composition (mol%) | Mean particle diameter (nm) | PDI | Nucleic acid enclosure rate (%) |
|---|---|---|---|---|---|
| 1 | TfR2 siRNA | DSPC:Chol:LP1:PEG2000-DMG=10:48:40:2 | 92 | 0.045 | 97 |
| 2 | A_Sin_GP1908_2015_H1 mRNA | DSPC:Chol:LP1:PEG2000-DMG=12.5:41:45:1.5 | 112 | 0.049 | 98 |
| 3 | A_Sin_GP1908_2015_H1 mRNA | DSPC:Chol:LP1:PEG2000-DMG=12.5:41:45:1.5 | 112 | 0.185 | 98 |
| 4 | A_Sin_GP1908_2015_H1 mRNA | DSPC:Chol:LP2:PEG2000-DMG=17.5:21:60:1.5 | 132 | 0.110 | 96 |

[Test Example 1] Test for evaluating drug efficacy of TfR2 siRNA-enclosed nucleic acid lipid particles in normal mouse

It has been reported that the TfR2 siRNA enclosed in the lipid particles in Example 1 and Reference Example 1 described above increases the plasma iron concentration in normal mice (WO 2012/177921). Therefore, the following test was conducted using the TfR2 siRNA-enclosed nucleic acid lipid particles (specimens 1 to 3) manufactured using a Taylor reactor and the TfR2 siRNA-enclosed nucleic acid lipid particles (Reference Example 1) manufactured by microchannel mixing.

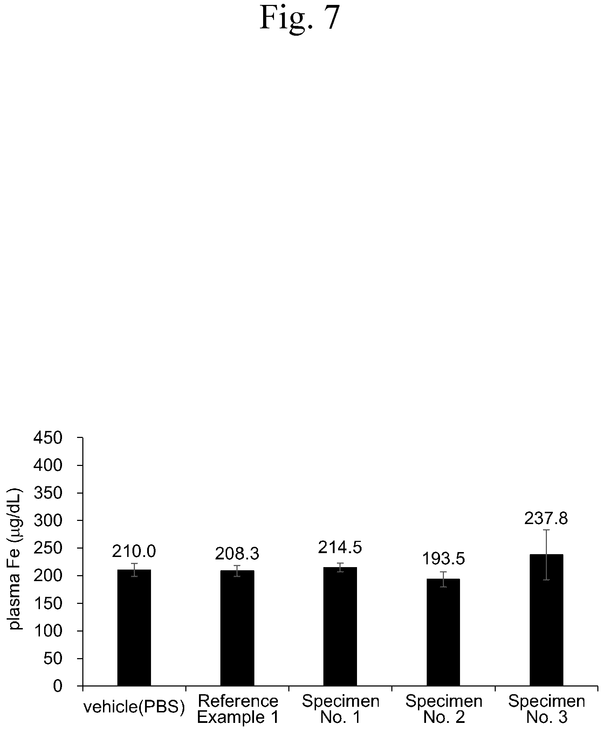

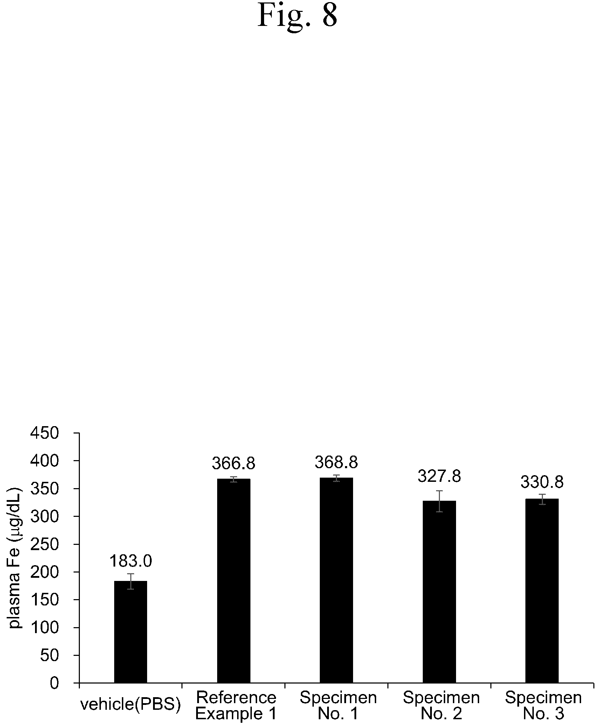

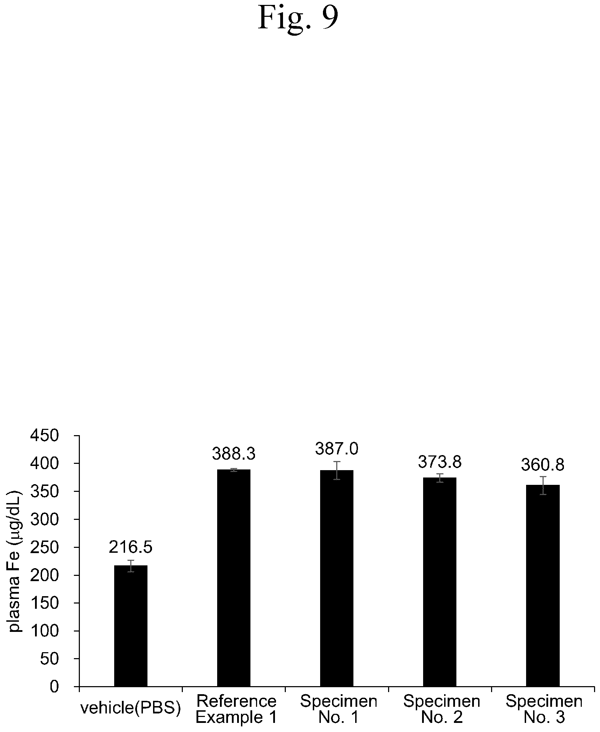

The TfR2 siRNA enclosed in the lipid particles was administered intravenously (i.v.) at a dose of 0.3 mg/kg (0.3 mpk) to male C57BL/6NJcl mice (manufactured by CLEA Japan, Inc.) (n = 4/group). As a vehicle group, PBS was administered intravenously (n = 4/group). Tail vein blood was collected before the treatment with siRNA or PBS (Pre), 1 day after the treatment (Day 1), and 7 days after the treatment (Day 7), and the plasma iron concentration (plasma Fe (ug/mL)) was measured using a metalloassay iron measurement kit (manufactured by Metallogenics Co., Ltd.). The results of the plasma iron concentrations are shown in Figs. 7 to 9 (mean ± standard error).

As shown in Figs. 7 to 9, 1 day and 7 days after the administration of the TfR2 siRNA-enclosed nucleic acid lipid particles, the effect of increasing the plasma iron concentration was observed as compared with the vehicle group. These results indicate that TfR2 siRNA-enclosed nucleic acid lipid particles manufactured using a Taylor reactor act in vivo.

[Test Example 2] Immunogenicity of monovalent LNP-mRNA: Production inducibility of HA-specific IgG of A Singapore GP1908 2015 H1 mRNA-enclosed nucleic acid lipid particles

In order to examine the in vivo immunity inducibility of the mRNA-enclosed nucleic acid lipid particle manufactured using the Taylor reactor, the following test was conducted using the A_Sin_GP1908_2015_H1 mRNA-enclosed nucleic acid lipid particles (specimens 9 to 14) and the A_Sin_GP1908_2015_H1 mRNA-enclosed nucleic acid lipid particles (Reference Examples 2 to 4) manufactured by microchannel mixing.

Into the gastrocnemius muscle of 7-week-old BALB/c mice, 0.2 μg of Reference Example 2, the specimen 9, the specimen 10, and the specimen 11 were administered twice at intervals of 2 weeks. Blood was collected 2 weeks after the last administration to prepare serum. Similarly, Reference Example 3, Reference Example 4, the specimen 12, the specimen 13, and the specimen 14 were administered to 6-week-old BALB/c mice to prepare serum. Specific IgG against HA in serum was detected by an Enzyme-linked immunosorbent assay (ELISA) method.

The immobilization treatment of the ELISA method was performed by adding 0.5 μg/mL of HA of recombinant A/Michigan/45/2015 to a 96 well plate at 25 μL/well and allowing the plate to stand at 4°C overnight. At the same time, in order to create a standard curve, mouse IgG at a known concentration was serially diluted and similarly immobilized. After removal of an immobilization liquid, the product was washed three times with DPBS containing 0.05% Tween 20 (DPBST), and subjected to blocking treatment with Dulbecco's phosphate buffered saline (DPBS) containing 0.05% Tween 20 and 1% bovine serum albumin (BSA) (DPBST/BSA). Sera were serially diluted with DPBST/BSA. After the blocking treatment, the product was washed three times with DPBST, and diluted serum was added at 25 μL/well. Instead of the diluted serum, DPBST/BSA was added to the well for the standard curve. The mixture was reacted at room temperature for 1 hour or more, then washed three times with DPBST, and horseradish peroxidase (HRP)-labeled anti-mouse IgG diluted with DPBST/BSA was added thereto. The mixture was reacted at room temperature for 1 hour, then washed three times with DPBST, and a 3,3',5,5'-tetramethylbenzidine (TMB) peroxidase substrate was added thereto at 30 μL/well. After allowing the mixture to stand at room temperature for about 10 minutes to develop color, a color development stopping liquid containing hydrochloric acid was added at 30 μL/well, and the absorbance at 450 nm was measured. The specific IgG concentration in the serum was calculated from the standard curve.

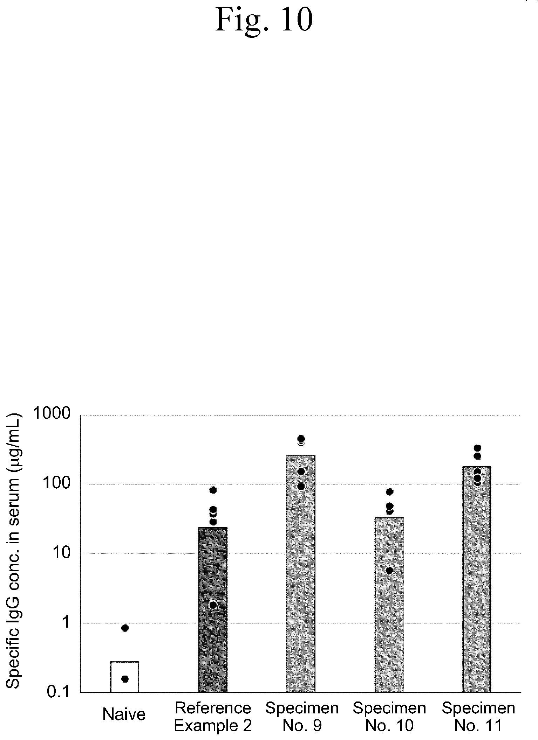

The results of administering Reference Example 2, the specimen 9, the specimen 10, and the specimen 11 are shown in Fig. 10. The specimen 10 showed the specific IgG induction level comparable to that of Reference Example 2, and the specimens 9 and 11 showed higher specific IgG induction levels than that of Reference Example 2.

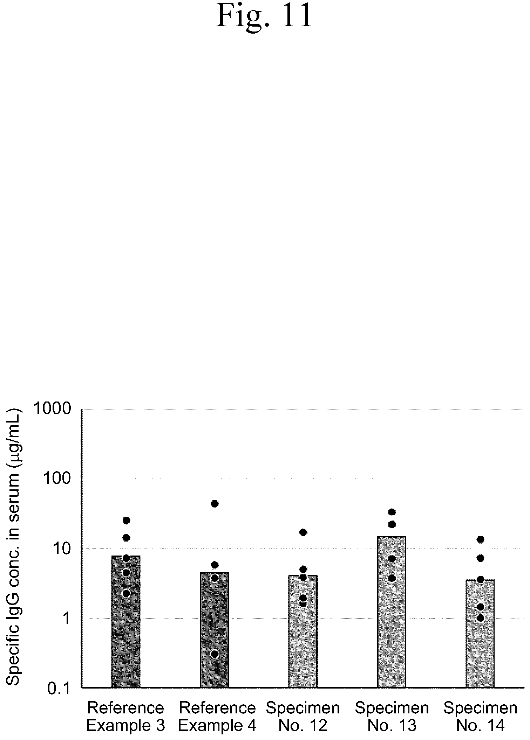

Similarly, the results of administering Reference Example 3, Reference Example 4, the specimen 12, the specimen 13, and the specimen 14 are shown in Fig. 11. Reference Examples 3 and 4 showed similar specific IgG induction levels. The specimens 12, 13, and 14 showed the specific IgG induction levels comparable to those of both Reference Examples.

These results indicate that A_Sin_GP1908_2015_H1 mRNA-enclosed nucleic acid lipid particles manufactured using a Taylor reactor induce the production of HA-specific IgG in vivo.

INDUSTRIAL APPLICABILITY

According to the present disclosure, a capsule particle can be manufactured. This can be used, for example, for the manufacture of RNA vaccines and siRNA medicines. In addition, the capsule particle can be continuously manufactured. In addition, the capsule particle can be manufactured on a large scale, but the present invention is not limited thereto.

Numerous documents are cited herein, including patent applications and manufacturer's manuals. The disclosures of these documents are not considered to be relevant to the patentability of the invention, but are incorporated herein by reference in their entirety. More particularly, all reference documents are to be incorporated herein by reference as if each document was specifically and individually indicated to be incorporated by reference. In the event of any discrepancy or inconsistency between the present specification and the cited document, the present specification shall control.

REFERENCE SIGNS LIST

1       Taylor reactor
2       outer cylinder
3       inner cylinder
4       inlet
4A      first line
4B      second line
4D      mixed liquid line
5       reaction chamber
5V      vortex row
6       outlet
7       controller
8       motor
11      first tank
12      first pump
13      second tank

14 second pump
15 third tank
16 third pump
20 jacket
21 chiller for controlling jacket temperature
41 first inlet
41A first line
41B second line
41D mixed liquid line
42 second inlet
43 third inlet
IR inner cylinder outer diameter
d gap width

**Claims**

1. A capsule particle manufacturing method using a Taylor reactor.

2. The manufacturing method according to claim 1, wherein

   the Taylor reactor comprises an outer cylinder, an inner cylinder, and one or more inlets,
   the inner cylinder is rotatably disposed in the outer cylinder and forms a reaction chamber having an annular shape between the inner cylinder and the outer cylinder, and
   at least one of the inlets is disposed so as to allow a fluid to flow into the reaction chamber.

3. The manufacturing method according to claim 2, wherein

   the one or more inlets include a first inlet and a second inlet, and the first inlet and the second inlet are independent of each other and are each connected to the reaction chamber,
   a particle-forming component is injected from the first inlet into the reaction chamber,
   an enclosed component is injected from the second inlet into the reaction chamber, and
   the inner cylinder is rotated to mix the particle-forming component and the enclosed component in the reaction chamber.

4. The manufacturing method according to claim 2, wherein

   the one or more inlets include a first line and a second line, the first line and the second line are disposed so as to merge, and a merged line is connected to the reaction chamber via a common inlet, and
   a particle-forming component is injected from the first line, an enclosed component is injected from the second line, the particle-forming component and the enclosed component are mixed in front of the reaction chamber to generate a particle-forming component-enclosed component mixed liquid, then the particle-forming component-enclosed component mixed liquid is injected into the reaction chamber via the common inlet, the inner cylinder is rotated, and the particle-forming component-enclosed component mixed liquid is further mixed in the reaction chamber.

5. The manufacturing method according to claim 3 or 4, wherein

   the particle-forming component comprises a polymer component or a lipid component, and
   the enclosed component comprises a nucleic acid, a protein, a polypeptide, a peptide, or a low molecular weight compound.

6. The manufacturing method according to claim 5, wherein

   the particle-forming component comprises a lipid component, and
   the enclosed component comprises a nucleic acid.

7. The manufacturing method according to claim 3, wherein the particle-forming component is injected first, and then the enclosed component is injected.

8. The manufacturing method according to claim 3, wherein the enclosed component is injected first, and then the particle-forming component is injected.

9. The manufacturing method according to claim 3, wherein the particle-forming component and the enclosed component are injected simultaneously.

10. The manufacturing method according to any one of claims 2 to 9, wherein a peripheral speed of the inner cylinder is 0.5 to 47 m/s.

11. The manufacturing method according to any one of claims 2 to 10, wherein a gap width between the inner cylinder and the outer cylinder is 0.01 mm to 5 mm.

12. The manufacturing method according to any one of claims 1 to 11, wherein a stirring time for performing a Taylor reaction is 1 minute or shorter.

13. The manufacturing method according to any one of claims 3 and 5 to 12, wherein a ratio of an injection rate of the particle-forming component to an injection rate of the enclosed component is a ratio selected from a range of 1 : 1 to 1 : 9.

14. The manufacturing method according to any one of claims 3 to 13, wherein inner diameters of the first inlet and the second inlet are the same or substantially the same.

15. The manufacturing method according to any one of claims 3 to 13, wherein inner diameters of the first inlet and the second inlet are different from each other.

16. A Taylor reactor for use in a capsule particle manufacturing method, the Taylor reactor comprising an outer cylinder, an inner cylinder, and one or more inlets, wherein

the inner cylinder is rotatably disposed in the outer cylinder and forms a reaction chamber having an annular shape between the inner cylinder and the outer cylinder, and
at least one of the inlets is disposed so as to allow a fluid to flow into the reaction chamber.

17. The reactor according to claim 16, wherein

the one or more inlets include a first inlet and a second inlet, and the first inlet and the second inlet are independent of each other and are each connected to the reaction chamber,
the first inlet is for injecting a particle-forming component,
the second inlet is for injecting an enclosed component, and
the inner cylinder is rotated for mixing the particle-forming component and the enclosed component in the reaction chamber.

18. The reactor according to claim 16, wherein

the one or more inlets have a first line and a second line,
the first line is for injecting a particle-forming component,
the second line is for injecting an enclosed component,
the first line and the second line merge in front of the reaction chamber,
a downstream side of a merging portion forms a particle-forming component-enclosed component mixed liquid line, which is then connected to the reaction chamber via a common inlet, and
the inner cylinder is rotated for further mixing a particle-forming component-enclosed component mixed liquid in the reaction chamber.

19. The reactor according to claim 17 or 18, wherein

the particle-forming component comprises a polymer component or a lipid component, and
the enclosed component comprises a nucleic acid, a protein, a polypeptide, a peptide, or a low molecular weight compound.

20. The reactor according to claim 19, wherein

the particle-forming component comprises a lipid component, and
the enclosed component comprises a nucleic acid.

21. The reactor according to claim 17 or claim 19 dependent on claim 17, wherein, when a side on which a liquid is supplied to the reaction chamber is defined as an upstream side and a side on which the liquid is discharged from the reaction chamber is defined as a downstream side, the first inlet is disposed on the upstream side and the second inlet is disposed on the downstream side.

22. The reactor according to claim 17 or claim 19 dependent on claim 17, wherein, when a side on which a liquid is supplied to the reaction chamber is defined as an upstream side and a side on which the liquid is discharged from the reaction chamber is defined as a downstream side, the second inlet is disposed on the upstream side and the first inlet is disposed on the downstream side.

23. The reactor according to claim 17 or claim 19 dependent on claim 17, wherein the first inlet and the second inlet are disposed on the same cross section perpendicular to an axial direction of the inner cylinder.

24. The reactor according to any one of claims 16 to 23, wherein a gap width between the inner cylinder and the outer cylinder is 0.01 mm to 5 mm.

25. The reactor according to any one of claims 17 and 19 to 24, wherein a ratio of an injection rate of the particle-forming component to an injection rate of the enclosed component is a ratio selected from a range of 1 : 1 to 1 : 9.

26. The reactor according to any one of claims 17 to 25, wherein inner diameters of the first inlet and the second inlet are the same or substantially the same.

27. The reactor according to any one of claims 17 to 25, wherein inner diameters of the first inlet and the second inlet are different from each other.

# Fig. 1

# Fig. 2

# Fig. 3

# Fig. 4

# Fig. 5

# Fig. 6

# Fig. 7

# Fig. 8

# Fig. 9

# Fig. 10

# Fig. 11

# EP 4 606 372 A1

## INTERNATIONAL SEARCH REPORT

| International application No. |
| --- |
| **PCT/JP2023/037566** |

### A. CLASSIFICATION OF SUBJECT MATTER

*A61K 9/48*(2006.01)i; *A61K 31/713*(2006.01)i; *A61K 38/02*(2006.01)i; *A61K 47/10*(2017.01)i; *A61K 47/18*(2017.01)i; *A61K 47/24*(2006.01)i; *A61K 47/28*(2006.01)i; *A61K 48/00*(2006.01)i; *A61P 7/00*(2006.01)i; *A61P 37/04*(2006.01)i; *B01J 19/22*(2006.01)i

FI: A61K9/48; A61K31/713; A61K48/00; A61K38/02; A61K47/24; A61K47/28; A61K47/18; A61K47/10; A61P7/00; A61P37/04; B01J19/22

According to International Patent Classification (IPC) or to both national classification and IPC

### B. FIELDS SEARCHED

Minimum documentation searched (classification system followed by classification symbols)

A61K9/48; A61K31/713; A61K38/02; A61K47/10; A61K47/18; A61K47/24; A61K47/28; A61K48/00; A61P7/00; A61P37/04; B01J19/22

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
| --- | --- | --- |
| X | KR 10-2013-0080539 A (SEOUL NATIONAL UNIVERSITY R&DB FOUNDATION) 15 July 2013 (2013-07-15) claim 1, paragraphs [0015], [0020], example 1, fig. 1 | 1-4, 7-18, 21-27 |
| A | | 5-6, 19-20 |
| X | US 2014/0120169 A1 (BOARD OF TRUSTEES OF MICHIGAN STATE UNIVERSITY) 01 May 2014 (2014-05-01) claims 1, 6, 8-9, example 2, fig. 1 | 1-27 |
| Y | | 1-27 |
| Y | JP 2020-532528 A (MODERNATX, INC.) 12 November 2020 (2020-11-12) claims 1-5 | 1-27 |
| Y | JP 2005-513145 A (CELATOR TECHNOLOGIES INC.) 12 May 2005 (2005-05-12) claims 1, 14-15 | 1-27 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **18 December 2023** | **09 January 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| International application No. |
| --- |
| **PCT/JP2023/037566** |

**C.    DOCUMENTS CONSIDERED TO BE RELEVANT**

| Category* | Citation of document, with indication, where appropriate, of the relevant passages | Relevant to claim No. |
| --- | --- | --- |
| Y | JP 2011-529912 A (ENZON PHARMACEUTICALS, INC.) 15 December 2011 (2011-12-15) claim 1 | 1-27 |

Form PCT/ISA/210 (second sheet) (January 2015)

**EP 4 606 372 A1**

## INTERNATIONAL SEARCH REPORT
### Information on patent family members

International application No.

**PCT/JP2023/037566**

| Patent document cited in search report | | | Publication date (day/month/year) | Patent family member(s) | | | Publication date (day/month/year) |
|---|---|---|---|---|---|---|---|
| KR | 10-2013-0080539 | A | 15 July 2013 | (Family: none) | | | |
| US | 2014/0120169 | A1 | 01 May 2014 | (Family: none) | | | |
| JP | 2020-532528 | A | 12 November 2020 | US<br>claims 1-5<br>WO<br>EP<br>CN | 2020/0306191<br><br>2019/046809<br>3675817<br>111315359 | A1<br><br>A1<br>A1<br>A | |
| JP | 2005-513145 | A | 12 May 2005 | US<br>claims 1, 14-15<br>WO<br>EP | 2003/0235619<br><br>2003/055469<br>1458365 | A1<br><br>A1<br>A1 | |
| JP | 2011-529912 | A | 15 December 2011 | US<br>claim 1<br>WO<br>EP | 2011/0111044<br><br>2010/014895<br>2304026 | A1<br><br>A2<br>A1 | |

Form PCT/ISA/210 (patent family annex) (January 2015)

# EP 4 606 372 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2022167048 A
- JP 2013536803 A
- WO 2015005253 A
- WO 2021060440 A
- WO 2020262150 A
- WO 2009127060 A
- WO 2012177921 A

### Non-patent literature cited in the description

- **SHAH et al.** Lipid Nanoparticles: Production, Characterization and Stability. Springer, 2015